# EUROPEAN PATENT APPLICATION

(11) **EP 4 151 646 A1**
(43) Date of publication of application: **22.03.2023**
(21) Application number: 21197835.8
(22) Date of filing: 20.09.2021
(51) Int. Cl.: C07H 19/10, A61P 35/00, A61K 31/7072

(54) **5-FLUOROURACIL DERIVATIVES AS PRODRUGS FOR CANCER TREATMENT**

(71) Applicant: Universität Hamburg, 20148 Hamburg (DE); Universitätsklinikum Hamburg-Eppendorf, 20246 Hamburg (DE)
(72) Inventor: Meier, Chris, 21635 Jork (DE); Schumacher, Udo, 22085 Hamburg (DE); Witt, Julian, 25436 Moorrege (DE); Roßmeier, Maria, 20537 Hamburg (DE); Remus, Simon, 20251 Hamburg (DE)
(74) Representative: Stüven, Ralf

(57) **Abstract**

The invention provides novel 5-Fluorouracil derivatives that are useful as prodrugs for cancer treatment. 5-Fluorouracil nucleoside triphosphates are masked at the terminal phosphate of the triphosphate group with residues that are cleaved off within the cell releasing the corresponding 5-Fluorouracil nucleoside triphosphate from the prodrug. In contrast to the classical prodrug 5-Fluorouracil, the 5-Fluorouracil derivatives of the invention need no further molecules (ribose, phosphate groups) to be added in the cell in order to become an active drug.

## Description

The invention relates to 5-Fluorouracil derivatives that are useful as prodrugs for cancer treatment.

Due to the lack of potent anti-cancer drugs a variety of different types of cancer represent a global threat to human health. In cancer chemotherapy so-called antimetabolites are often used as cytostatic drugs. Antimetabolites are very similar in structure to the body's own metabolites that are needed in cell division and cell growth, but differ from them by characteristic groups in such a way that they have a cytotoxic effect. An important class of antimetabolites are nucleoside analogues, containing, for example, a nucleobase analogue such as 5-fluorouracil (5-FU), i.e. a nucleobase different from but similar to the cell's own nucleobases. The aim of this medication is that these base analogues are incorporated into the target cells and used there instead of the cell's own substances to replicate or elongate the DNA and RNA. As a consequence, DNA strand breaks, unstable molecules or a stoppage of the elongation occurs, all of which ultimately lead to cell death.

However, the efficiency of nucleoside analogues is limited by various cellular barriers. First, their uptake is largely dependent on human nucleoside transporters. Second, once inside the cell, the metabolic conversion of nucleoside analogues into their ultimately active triphosphates often proceeds insufficiently resulting in a low activity of the drug.

A long-known representative of such an antimetabolite used in cancer chemotherapy is a uracil, substituted with a fluorine atom on the carbon in the C5 position (5-fluorouracil, 5-FU). This is a prodrug which is only converted into an active drug by metabolism in the cancer cell. Since 5-FU is an analogue of a nucleobase, it still has to be combined with a ribose and three phosphates at the 5'-position of the ribose ring in the cancer cell to finally serve as an adequate substrate for DNA or RNA synthesis. Key enzyme for these steps is the enzyme uridine monophosphate synthetase (UMPS).

The cytotoxicity of 5-FU is the result of two different effects. First, in cells with sufficiently high UMPS expression, it serves as a substrate for the orotate phosphoribosyl transferase (OPRT) domain of UMPS, which converts 5-FU into a monophosphate. It can now be further phosphorylated and thus incorporated into the RNA as F-UTP. However, the resulting RNA strand is not useful for the cell, which interferes with the translation process and other effects of various RNAs of the target cell. Furthermore, 5-FU can be further modified to become F-dUMP, a substrate of thymidylate synthase. This results in irreversible binding to the active site of this enzyme. The enzyme molecule is then completely inactivated. This is called a suicide substrate mechanism. These two effects combined lead to a high cytotoxicity which is the reason why 5-FU is a very effective cytostatic drug.

However, there are also serious disadvantages. Since 5-FU is so strongly dependent on target cell enzymes for its activation to an active drug, and the affinity of these enzymes is significantly higher for the cell's own metabolites than for 5-FU, high drug concentrations are necessary when using 5-FU in chemotherapy. This makes the therapy very stressful for the entire organism. In order to contain these unwanted effects and to make 5-FU more targeted, it is nowadays almost exclusively used in combination therapies with other substances (FOLFOX - together with folinic acid and oxaliplatin - or FOLFIRI - with folinic acid and irinotecan).

It is known that nucleoside analogues cannot penetrate the cell membrane under physiological conditions in the form of their mono-, di- or triphosphates (NMP, NDP, NTP), due to their charge. Therefore, nucleoside analogues must either be intracellularly converted into their triphosphates by more or less specific kinases in several stages, or phosphorylated nucleoside analogues must be designed or modified so as to be able to pass through the cell membrane. WO 2009/129798 A2, WO 2016/026493 A1 and WO 2018/100137 A1, for example, describe nucleoside triphosphate prodrugs, in particular for use in antiviral therapy, where the nucleoside triphosphate is masked at the terminal phosphates with lipophilic masks allowing the NTP prodrug to enter the cell.

It is an object of the invention to provide a means for a more efficient use of the known cytostatic compound 5-fluorouracil for cancer treatment.

The problem is solved according to the invention by a 5-Fluorouracil nucleoside triphosphate prodrug having the following general formula I 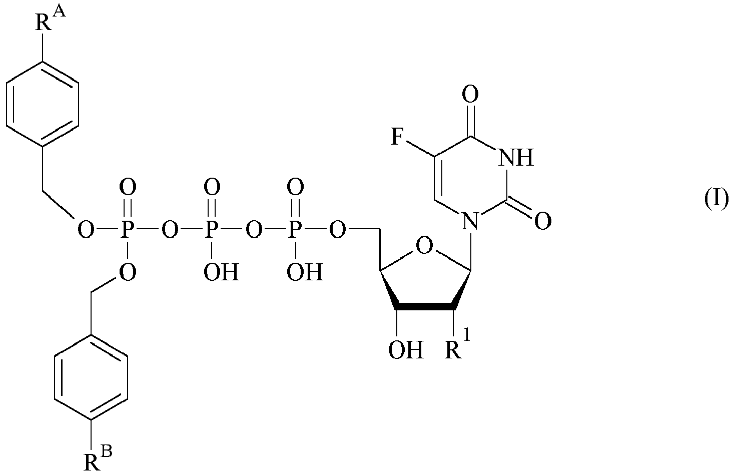 or a pharmaceutically acceptable salt thereof, wherein
R¹ is H or OH,
R^{A} is H or OC(X)R^{A1}, wherein X is O, S or NH,
R^{B} is H or OC(X)R^{B1}, wherein X is O, S or NH,
R^{A} and R^{B} are not both H, and
R^{A1} and R^{B1} are, independently from each other, a substituted or unsubstituted cyclic, acyclic, linear or branched aliphatic or heteroaliphatic residue, or a substituted or unsubstituted aromatic or heteroaromatic residue.

The invention provides a single or, preferably, a double bioreversible masked 5-Fluorouracil nucleoside triphosphate intracellular delivery system that can advantageously be used as an anti-cancer antimetabolite. Such a concept was recently successful in the arena of antivirally active nucleoside analogues. The 5-FU NTP prodrug according to the invention enables both an improved cellular uptake by passive diffusion as well as a bypass of all enzymatic steps for conversion of the prodrug 5-Fluorouracil into an active drug by the direct intracellular delivery of the NTP. Therefore, a significant higher concentration of the active triphosphorylated agent inside tumor cells can be accomplished with the consequence of a better drug activity. The invention involves the symmetric or non-symmetric chemical modification of 5-FU nucleoside triphosphate at the terminal phosphate group containing the γ-phosphorus atom by covalently attaching two intracellularly labile residues (masks) that are removed enzymatically within the cell.

The invention provides 5-FU nucleoside triphosphate (5-FU NTP) prodrugs, which are converted intracellularly into the corresponding 5-FU nucleoside triphosphate. By means of the masking according to the invention of the terminal phosphate group counter-acting the negative charge of this phosphate group of the triphosphate, 5-FU nucleoside triphosphate can be successfully introduced into the cell and released from the prodrug as active agent. There is no need for additional masking of the internal phosphate(s). The two masks themselves are also not charged under physiological conditions.

Both masks of a double bioreversible masked 5-Fluorouracil nucleoside triphosphate of the invention are intracellularly labile, for example enzymatically cleavable, e.g. by means of esterases, so that after e.g. enzymatic cleavage of the masks within the cell free 5-FU nucleoside triphosphate results. Preferably, at least one of the masks, particular preferred both masks, are lipophilic and thus ensure the necessary lipophilicity in order to ensure entry into the cell. The adjustment of the lipophilicity of a mask is known to a person skilled in the art and can, if necessary, be determined by routine experimentation. For example, the lipophilicity of a mask can be manipulated by the size or length of a hydrocarbon residue.

The approach of the invention, i.e. the use of a lipophilic, membrane permeable prodrug of the ultimately antitumor-active 5-FU nucleoside triphosphate instead of the nucleobase analogue 5-FU, allows a lower molecular concentration of the drug to reach IC₅₀. Furthermore, these derivatives bypass several enzymes, which convert the inactive 5-FU into an active drug. The down-regulation of one or more of these enzymes has been shown to occur in many 5-FU resistant cases. Thereby the 5-FU derivatives of the invention can also be used, for example, as an ideal second line therapy in 5-FU resistant cases. In addition, the therapeutic index is broader with the triphosphate prodrug of the invention in comparison to 5-FU.

Under physiological conditions, e.g. within a cell, the compounds according to the invention are generally present as salts. In the following formula Ia cations are symbolized with a plus sign within a circle. Cations may, for example, be ammonium, sodium or potassium cations. Further, the alpha-, beta- and gamma-phosphates are indicated.

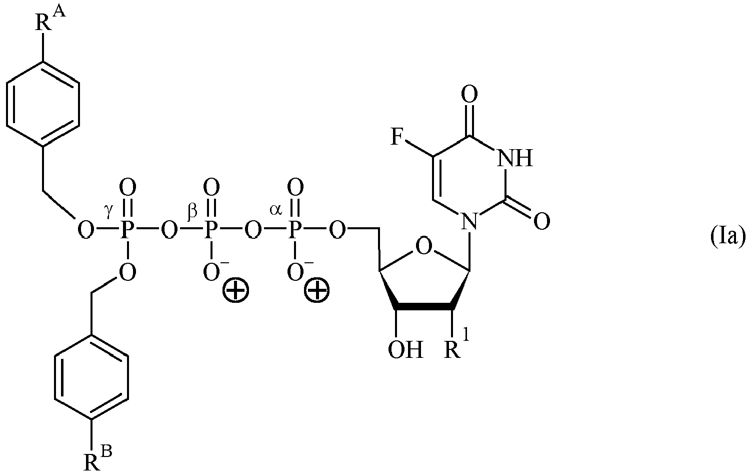

The term "5-Fluorouracil nucleoside triphosphate" ("5-FU nucleoside triphosphate", "5-FU NTP") relates to a compound according to the below formula 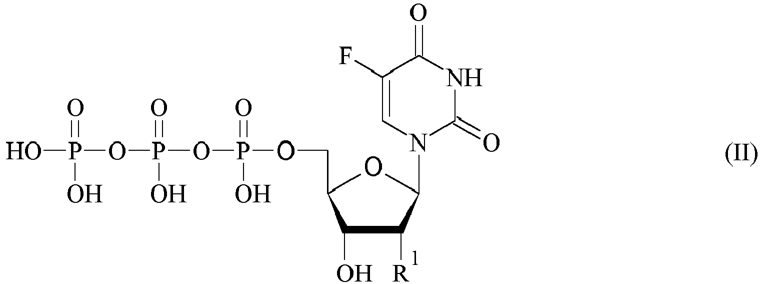 wherein R¹ is H or OH, or any salt thereof. The term thus relates to a nucleoside triphosphate having 5-Fluorouracil as a nucleobase covalently bound to C₁ of ribose or deoxyribose. The term thus encompasses the terms "5-fluorouridine triphosphate" and "5-fluorodeoxyuridine triphosphate". The term "5-Fluorouracil nucleoside analogue triphosphate" may be used synonymously with the term "5-Fluorouracil nucleoside triphosphate".

The term "prodrug" relates to a precursor of a drug, i.e. a compound, which itself is pharmacologically inactive or at least only little active, and undergoes chemical conversion by metabolic processes before becoming an active pharmacological agent.

The term "pharmaceutically acceptable salt" means any salt of a compound that is substantially non-toxic for mammals, especially humans, and that does not substantially affect the effectiveness of the biological activity of the active ingredient.

The term "nucleoside triphosphate or nucleoside triphosphate analogue" refers to nucleoside triphosphates or analogues thereof, a nucleoside triphosphate being a compound according to the following general formula III 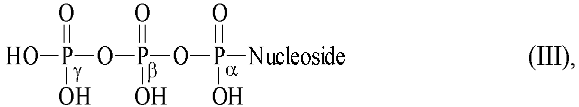 and a "nucleoside triphosphate analogue" being an analogue of a nucleoside triphosphate. Nucleoside triphosphates are composed of a base component (nucleobase), a sugar component, e.g. a pentose like ribose or deoxyribose, and a triphosphate residue (sometimes also termed "triphosphate bridge") attached to an O atom of the sugar component, e.g. an O atom bound to the 5' atom of a pentose. A "nucleoside triphosphate analogue" is a chemical compound, which is structurally and/or functionally similar to a nucleoside triphosphate such that an enzyme, e.g. a polymerase, having a nucleoside triphosphate naturally occurring in, for example, a human cell as a substrate would also use said compound as a substrate, analogous to the naturally occurring nucleoside triphosphate. 5-fluorouridine triphosphate is an example of a nucleoside analogue being modified in the base component, compared to a naturally occurring nucleoside triphosphate.

The term "terminal phosphate group" refers to the terminal or γ-phosphate (γ-phosphoryl) group of the triphosphate group. The term "γ-phosphorus atom" as used herein relates to the phosphorus atom of the terminal phosphorus containing group of the triphosphate component.

The term "non-symmetrical" in relation to the modification or masking of 5-Fluorouracil nucleoside triphosphate at γ-phosphate is understood to mean that the nucleoside triphosphate is twice, but non-symmetrically, modified at the γ-phosphorus atom thereof, by covalently attaching two different lipophilic groups. In particular, the term means that the residues R^{A} and R^{B} are different from each other. The term "symmetrical" correspondingly means that the two masking groups are identical in structure, in particular that the residues R^{A} and R^{B} are identical in structure.

An "intracellularly labile residue", which may also be referred to as "intracellularly labile mask", or as an "intracellularly labile group", "enzymatically cleavable moiety", "enzymatically cleavable group", or the like, is understood here to be a chemical group which is, e.g. hydrolytically, cleavable under conditions prevailing in a target cell, preferably a eukaryotic, for example human cell, for example in terms of temperature, pH, salt content, etc., with the aid of enzymes present or, as the case may be, inducible in the target cell. The term also encompasses photolabile, i.e. photocleavable moieties, e.g. groups that can be cleaved by irradiation with UV or near-UV light. The term also encompasses those cases in which a mask is degraded in several steps, for example cascade-like, and at least one degrading step takes place enzymatically. For example, the initial attack can be carried out enzymatically, while the further degradation steps occur spontaneous. In particular, a residue is considered to be intracellularly labile, if the compound masked therewith has a comparatively low half-life in the cell, for example a half-life of ≤ 4 h, preferably ≤ 3 h, ≤ 2 h, ≤ 1 h, ≤ 45 min or ≤ 30 min.

By "nucleoside" are meant organic molecules consisting of a sugar residue (sugar component) linked to an organic base (base component), e.g. a heterocyclic organic base, in particular a nitrogen-containing heterocyclic organic base (nucleobase), which are linked via a glycosidic bond. The sugar moiety is often a pentose, e.g. deoxyribose or ribose, but may also be another sugar, e.g. a C₃-, C₄- or C₆-sugar. Frequently, but not exclusively, nucleobases are purines (R) or pyrimidines (Y). Examples of naturally occurring purines are guanine (G) and adenine (A), examples of naturally occurring pyrimidines are cytosine (C), thymine (T) and uracil (U). In particular, a nucleoside is therefore a compound of the following general formula 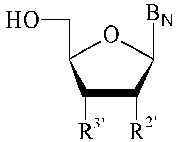 wherein B_{N} is a nitrogen-containing heterocyclic organic base, e.g. a nucleobase, and R^{2'} and R^{3'} are, independently from each other, H or OH. Phosphorylated nucleosides, for example nucleoside monophosphates (NMP), nucleoside diphosphates (NDP) and nucleoside triphosphates (NTP), are also referred to as nucleotides. The phosphate, diphosphate (pyrophosphate) or triphosphate group is generally linked to the O atom attached to the 5'-C atom of the sugar component of the nucleoside.

A "nucleoside analogue" (or "nucleoside analog") is to be understood here as an organic compound which is naturally not present in the human body but which is structurally similar to a nucleoside occurring naturally in the human body so that it can be used, for example, by the cell enzymes essentially corresponding to the natural nucleoside, for example phosphorylated and incorporated into an RNA or DNA strand.

A "nucleoside phosphate analogue" is understood to mean an analogue to a phosphorylated nucleoside, i.e. a nucleotide analogue. The term "nucleoside monophosphate analogue" is, for example, understood to mean an analogue to a nucleoside monophosphate.

The terms "acyloxybenzyl residue", "acyloxybenzyl group" or "acyloxybenzyl mask", may be used for a residue of the following general structure 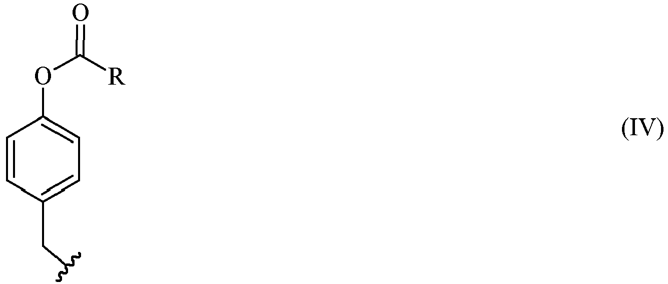 wherein R may be, for example, R^{A1} or R^{B1}.

The term "Tri*PPP*ro compound" is used herein in relation to nucleoside triphosphate or nucleoside triphosphate analogue prodrugs, i.e. nucleoside triphosphates or nucleoside triphosphate analogues suitably masked at the terminal phosphate group of the triphosphate group (see, for example, Gollnest, T. 2015, Das TriPPPro-Konzept: Entwicklung und Charakterisierung von antiviralen Nukleosidtriphosphat-Prodrugs, Dissertation, Hamburg University, urn:nbn:de:gbv: 18-78516).

For the purposes of the present invention, the indication of a range such as "1-10", for example, is to be understood as meaning that each intermediate value is also disclosed. In the case of an indication which can only affect integers, such as, for example, a number of C atoms, this means, of course, that only integers are disclosed. Any narrower range from a broader range is also meant to be disclosed by indicating the broader range, the narrower range also including ranges not comprising any of the boundary values of the broader range (e.g. a range of 2-5 from a range of 1-10).

The term "Cₙ-Cₘ" or "Cₙ₋ₘ", where n and m are each positive integers and m is greater than n, means a range indicating the number of carbon atoms of a compound or residue. The expression here expressly includes all integer intermediate values between the range boundaries n and m, in each case independently of one another. The expression "C₁₋₁₀" (n = 1, m = 10) therefore means, for example, a compound, group or residue having 1-10, i.e. 1, 2, 3, 4, 5, 6, 7, 8, 9 or 10 carbon atoms. "C₁₋₁₀" therefore also comprises, for example, "C₂₋₆", i.e. 2, 3, 4, 5 or 6 carbon atoms, or "C₁₋₄", i.e. 1, 2, 3 or 4 carbon atoms, or "C₄₋₉", i.e. 4, 5, 6, 7, 8 or 9 carbon atoms. Correspondingly, the term "C₁₋₂₀-alkyl" means, for example, an alkyl group having 1, 2, 3, 4, 5, 6, 7, 8, 9, 10, 11, 12, 13, 14, 15, 16, 17, 18, 19 or 20 carbon atoms and comprises all combinations of the values of n and m, which lie in the range from n = 1 to m = 20, for example "C₁₋₁₀ alkyl", i.e., an alkyl having 1, 2, 3, 4, 5, 6, 7, 8, 9 or 10 carbon atoms, or "C₅₋₇ alkyl", i.e. an alkyl having 5, 6 or 7 carbon atoms. Correspondingly, this also applies to terms such as "C₂₋₁₀ alkenyl", "C₄₋₂₀ alkenynyl", and the like.

The term "aliphatic residue" comprises cyclic or acyclic linear (straight chain) or branched, saturated or unsaturated carbon compound residues, other than aromatic residues. The term "heteroaliphatic residue" means aliphatic residues in whose carbon skeleton one or more C atoms are replaced by heteroatoms, for example oxygen, sulfur, nitrogen or phosphorus.

The term "alkyl" comprises saturated aliphatic (non-aromatic) groups including straight-chain (linear) alkyl groups (e.g. methyl, ethyl, propyl, butyl, pentyl, hexyl, heptyl and octyl), and branched alkyl groups (e.g. isopropyl, tert-butyl, isobutyl). The term also encompasses O, N, S or P alkyl groups (e.g., -O-methyl), i.e. alkyl groups which are bound to a compound via an oxygen, nitrogen, sulfur or phosphorus atom.

The term "alkenyl" comprises unsaturated aliphatic (non-aromatic) groups having at least one C-C double bond, including straight-chain and branched alkenyl groups. The term also includes O-, N-, S- or P-alkenyl groups (e.g., -O-propenyl), i.e. alkenyl groups bound to a compound via an oxygen, nitrogen, sulfur or phosphorus atom.

The term "alkynyl" (or "alkinyl") includes unsaturated aliphatic (non-aromatic) groups having at least one C-C triple bond, including straight-chain and branched alkynyl groups. The term also includes O, N, S or P alkynyl groups (e.g., -O-butinyl), i.e. alkynyl groups bound to a compound via an oxygen, nitrogen, sulfur or phosphorus atom.

The term "alkenynyl" (or "alkeninyl") includes unsaturated aliphatic (non-aromatic) groups having at least one C-C double bond and at least one C-C triple bond, including straight-chain and branched alkenynyl groups. The term also includes O-, N-, S-, or P-alkenynyl groups, i.e. alkenynyl groups bound to a compound via an oxygen, nitrogen, sulfur or phosphorus atom.

The term "cycloalkyl" includes compounds containing an alicyclic group, i.e. a ring-shaped saturated aliphatic (non-aromatic) group, e.g. cyclopropyl, cyclopentyl, cyclohexyl, cycloheptyl, cyclooctyl group. The term also includes O-, N-, S- or P-cycloalkyl groups, i.e. cycloalkyl groups bound to a compound via an oxygen, nitrogen, sulfur or phosphorus atom. The terms "cycloalkenyl", "cycloalkynyl" and "cycloalkenynyl" mean correspondingly ring-shaped aliphatic (non-aromatic) alkenyls, alkynyls or alkenynyls as defined above, the double and/or triple bond(s) being present within or outside the ring or ring system.

The term "heteroalkyl" refers to alkyl groups in which one or more carbon atoms of the hydrocarbon structure are replaced by other atoms (heteroatoms), e.g. oxygen, nitrogen, sulfur or phosphorus atoms. The term also includes O-, N-, S- or P-heteroalkyl groups, i.e. heteroalkyl groups which are bound to a compound via an oxygen, nitrogen, sulfur or phosphorus atom. The term "heteroalkyl" also includes cycloalkyls in which one or more carbon atoms of the hydrocarbon structure are replaced by other atoms, e.g. oxygen, nitrogen, sulfur or phosphorus atoms. The terms "heteroalkenyl", "heteroalkynyl", "heteroalkeninyl" mean corresponding alkenyls, alkynyls and alkeninyls, as well as cycloalkenyls, cycloalkynyls and cycloalkeninyls, in which one or more carbon atoms of the hydrocarbon skeleton are replaced by other atoms (heteroatoms), e.g. oxygen, nitrogen, sulfur or phosphorus atoms. For example, the term "C₁₋₂₀-heteroalkyl" means an alkyl group having 1, 2, 3, 4, 5, 6, 7, 8, 9, 10, 11, 12, 13, 14, 15, 16, 17, 18, 19 or 20 carbon atoms and at least one heteroatom. The same also applies to heteroalkenyls, heteroalkynyls and heteroalkenynyls.

By "aryl" are meant groups with aromaticity, including multi-membered aromatic single ring groups and multicyclic systems with at least one aromatic ring. Examples of aryl groups include benzene, phenyl and naphthalene. The term also includes O-, N-, S- or P-aryl groups, i.e. aryl groups bound to a compound via an oxygen, nitrogen, sulfur or phosphorus atom.

By "heteroaryl" are meant aryl groups having at least one heteroatom in the ring structure, i.e. in which one or more carbon atoms in the ring structure are replaced by other atoms (heteroatoms), e.g. b oxygen, nitrogen, sulfur or phosphorus atoms. Examples of heteroaryls are pyrrole, furan, thiophene, thiazole, isothiazole, imidazole, triazole, tetrazole, pyrazole, oxazole, pyridine, pyrazine, pyridazine and pyrimidine. The term also includes multicyclic, e.g. bicyclic and tricyclic, aryl groups, e.g. benzoxazole, benzodioxazole, benzothiazole, benzoimidazole, benzothiophene, methylenedioxyphenyl, quinoline, isoquinoline, indole, benzofuran, purine or benzofuran. The term also includes O-, N-, S- or P-heteroaryl groups, i.e. heteroaryl groups bound to a compound via an oxygen, nitrogen, sulfur or phosphorus atom.

By the term "halogen" is meant chlorine (Cl), fluorine (F), bromine (Br) and iodine (I), in particular chlorine (Cl) and fluorine (F).

The term "substituted" means that one or more substituents are present, which replace a hydrogen atom on one or more carbon atoms of the hydrocarbon structure or on one or more heteroatoms in the carbon skeleton. Examples of such substituents are oxo, hydroxyl, phosphate, cyano, azido and amino groups, but also e.g. halogens (e.g., F, Cl, Br), acyl, acyloxy, alkyl, cycloalkyl, heteroalkyl, heterocycloalkyl, aryl and heteroaryl groups. Instead of the term "substituent" also the terms "residue" or "moiety" may be used here.

The terms "triple-negative breast cancer" (TNBC), "triple negative breast carcinoma" or "triple-negative mamma carcinoma" relate to a type of breast cancer that is characterised by tumor cells that are deficient in estrogen, progesterone and ERBB2 (HER2) receptor expression (see, for example, Medina, M. A., Oza, G., Sharma, A., Arriaga, L. G., Hernandez Hernandez, J. M., Rotello, V. M., & Ramirez, J. T. (2020). Triple-Negative Breast Cancer: A Review of Conventional and Advanced Therapeutic Strategies. International journal of environmental research and public health, 17(6), 2078, doi:10.3390/ijerph17062078; Gupta, G.K.; Collier, A.L.; Lee, D.; Hoefer, R.A.; Zheleva, V.; Siewertsz van Reesema, L.L.; Tang-Tan, A.M.; Guye, M.L.; Chang, D.Z.; Winston, J.S.; Samli, B.; Jansen, R.J.; Petricoin, E.F.; Goetz, M.P.; Bear, H.D.; Tang, A.H. Perspectives on Triple-Negative Breast Cancer: Current Treatment Strategies, Unmet Needs, and Potential Targets for Future Therapies. Cancers 2020, 12, 2392, doi:10.3390/cancers12092392). The cell line MDA-MB231 is a triple-negative breast cancer cell line (see, e.g., Chavez, K. J., Garimella, S. V., & Lipkowitz, S. (2010). Triple negative breast cancer cell lines: one tool in the search for better treatment of triple negative breast cancer, Breast disease, 32(1-2), 35-48, doi:10.3233/BD-2010-0307).

The term "colon cancer" or "colon carcinoma" relate to colorectal cancer (CRC), i.e. a cancer of the colon or rectum. SW620, HCT116, HT29 and CaCo2 (Caco-2) are colon cancer cell lines (see, for example, Ahmed, D., Eide, P., Eilertsen, I. Danielsen S.A., Eknæs M., Hektoen M., Lind G.E., Lothe R.A., Epigenetic and genetic features of 24 colon cancer cell lines. Oncogenesis 2, e71 (2013), doi:10.1038/oncsis.2013.35; Hidalgo IJ, Raub TJ, Borchardt RT. (1989) Characterization of the human colon carcinoma cell line (Caco-2) as a model system for intestinal epithelial permeability, Gastroenterology 96:736-749, doi:10.1016/0016-5085(89)90897-4; Lea T. (2015) Caco-2 Cell Line. In: Verhoeckx K. et al. (eds) The Impact of Food Bioactives on Health. Springer, Cham. doi:10.1007/978-3-319-16104-4_10).

It is to be understood that the residues OC(X)R^{A1} or OC(X)R^{B1}, are attached via the oxygen atom to the phenyl moiety, as shown below as an example:

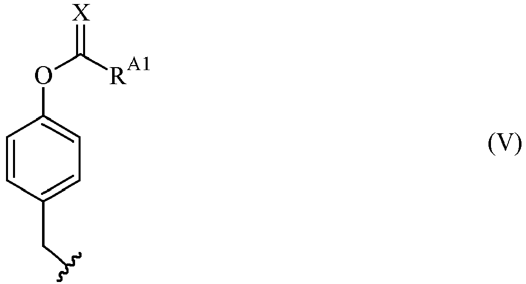

In a preferred embodiment of the 5-Fluorouracil nucleoside triphosphate prodrug of the invention, R^{A1} and R^{B1} are, independently from each other,
i. a substituted or unsubstituted cyclic, acyclic, linear or branched C_{1―20} aliphatic or C_{1―20} heteroaliphatic residue, or a substituted or unsubstituted C_{5―20} aromatic or C_{3―20} heteroaromatic residue, or
ii. a substituted or unsubstituted cyclic, acyclic, linear or branched C_{1―10} aliphatic or C_{1―10} heteroaliphatic residue, or a substituted or unsubstituted C_{5―12} aromatic or C_{3―12} heteroaromatic residue, or
iii. selected from the group consisting of substituted or unsubstituted C_{1―20} alkyl, substituted or unsubstituted C_{2―20} alkenyl, substituted or unsubstituted C_{2―20} alkynyl, substituted or unsubstituted C_{4―20} alkenynyl, substituted or unsubstituted C_{3―20} cycloalkyl, substituted or unsubstituted C_{3―20} cycloalkenyl, substituted or unsubstituted C_{5―20} cycloalkynyl, substituted or unsubstituted C_{5―20} cycloalkenynyl, substituted or unsubstituted C_{1―20} heteroalkyl, substituted or unsubstituted C_{2―20} heteroalkenyl, substituted or unsubstituted C_{2―20} heteroalkynyl, substituted or unsubstituted C_{4―20} heteroalkenynyl, substituted or unsubstituted C_{5―24} aryl, substituted or unsubstituted C_{3―24} heteroaryl, preferably C_{1―20} alkyl or C_{1―20} alkenyl.

In a preferred embodiment of the invention the residues R^{A} and R^{B} are identical. In another preferred embodiment of the invention the residues R^{A} and R^{B} are different from each other. In further preferred embodiments of the invention the residue R^{A} is OC(O)XR^{A1} and the residue R^{B} is OC(O)XR^{B1}, wherein the residues R^{A1} and R^{B1} are as defined herein, and may be identical or different.

In a preferred embodiment of the 5-Fluorouracil nucleoside triphosphate prodrug according to the invention R¹ is H or OH, R^{A} is OC(O)XR^{A1} and R^{B} is OC(O)XR^{B1}, wherein X is O, R^{A1} is C₅₋₁₅-alkyl, preferably C₈₋₁₃ alkyl, and R^{B1} is C₅₋₁₅ alkyl, preferably C₈₋₁₃ alkyl. In a further preferred embodiment of the 5-Fluorouracil nucleoside triphosphate prodrug according to the invention R¹ is H, R^{A} is OC(O)XR^{A1} and R^{B} is OC(O)XR^{B1}, wherein X is O, R^{A1} is C₅₋₁₅-alkyl, preferably C₈₋₁₃ alkyl, and R^{B1} is C₅₋₁₅ alkyl, preferably C₈₋₁₃ alkyl. Preferred embodiments of the compounds of the invention thus have, for example, the following structure of general formula (Ib) 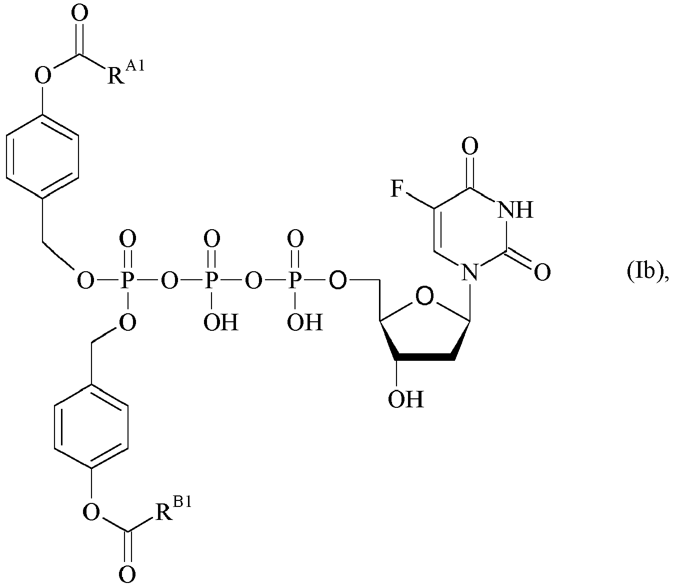 wherein R^{A1} is C₅₋₁₅-alkyl, preferably C₈₋₁₃ alkyl, and R^{B1} is C₅₋₁₅ alkyl, preferably C₈₋₁₃ alkyl. Particularly preferred, R^{A1} and R^{B1} are structurally identical.

Particularly preferred, residue R^{A1} is C₈ alkyl or C₁₁ alkyl, and R^{B1} is C₈ alkyl or C₁₁ alkyl. Preferably, R^{A1} and R^{B1} are identical, i.e. in case R^{A1} is C₈ alkyl, R^{B1} is also C₈ alkyl, and in case R^{A1} is C₁₁ alkyl, R^{B1} is also C₁₁ alkyl.

An example of one of the particularly preferred compounds of the invention is presented below for illustration purposes with formula Ib1:

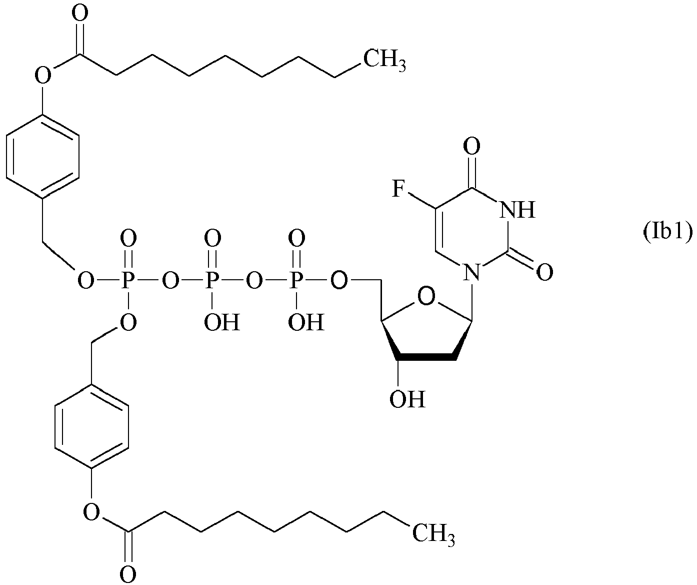

In this example, both residues R^{A1} and R^{B1} are C₈ alkyl.

Another example of a preferred compound of the invention is depicted below for illustration purposes with formula Ib2:

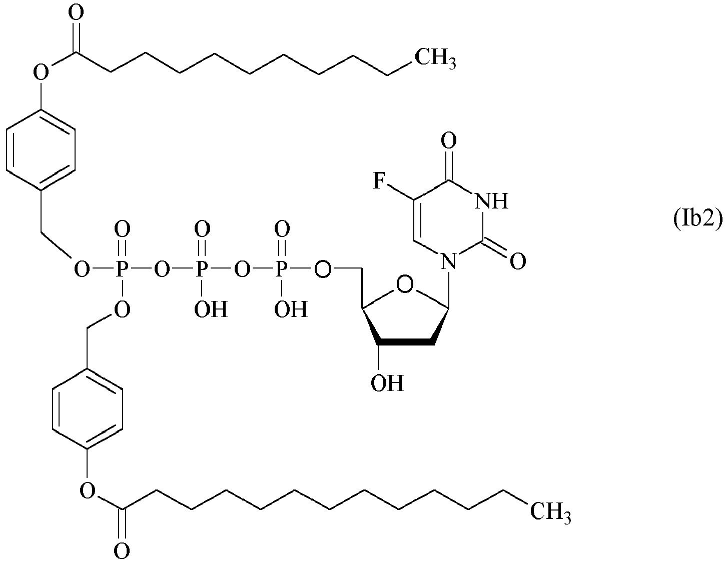

Here, the compound is asymmetrically substituted, i.e. residues R^{A1} and R^{B1} are different from each other, and are C10 (R^{A1}) and C12 alkyl (R^{B1}).

The compounds according to the invention exhibit good cell penetrability and intracellularly release of the final active pharmaceutical agent 5-Fluorouracil nucleoside triphosphate. The prodrugs according to the invention are thus suitable for use as a medicament, in particular as medicament for treating cancer, for example, colon carcinoma, breast cancer or other cancers that are susceptible to 5-FU, like esophageal cancer, head and neck cancer, stomach cancer, anal cancer or squamous cell carcinoma. In relation to breast cancer, the 5-Fluorouracil nucleoside triphosphate prodrug of the invention is particularly useful as a medicament for treating triple negative breast cancer (TNBC).

The invention therefore also relates to a pharmaceutical composition or dosage form comprising a compound according to the invention and a pharmaceutically acceptable carrier. Pharmaceutically acceptable carriers are known to the person skilled in the art and may comprise one or more liquid, semi-solid or solid fillers, diluents or other substances suitable for administration to mammals, including humans.

For the purposes of the present invention, the term "carrier" refers to any organic or inorganic, natural or synthetic substance which can be combined with the active ingredient to simplify application. Examples of such carriers include, but are not limited to organic or inorganic solvents, starch, lactose, mannitol, methylcellulose, talc, gelatin, agar agar, calcium phosphate, magnesium stearate, animal and vegetable fats, higher molecular weight fatty acids, lipids, phospholipids, or higher molecular weight polymers.

The term "pharmaceutically acceptable" means any substantially non-toxic material for mammals, especially humans, which does not substantially affect the effectiveness of the biological activity of the active ingredient. Such materials may include pharmaceutically acceptable concentrations of salts, buffers, preservatives, or the like. Non-limiting examples of pharmaceutically acceptable carriers include magnesium carbonate, magnesium stearate, talc, sugar, lactose, ethanol, glycerol, water, buffer solutions. The pharmaceutical composition may also comprise adjuvants and/or diluents.

The pharmaceutical composition or dosage form according to the invention may be adapted to any suitable route of administration, e.g. for oral, rectal or parenteral administration. A preferred pharmaceutical composition or dosage form of the invention is a adapted for oral administration, for example a tablet or capsule. In a further preferred embodiment of the invention the pharmaceutical composition or dosage form is a liposome or liposome formulation (see, for example, Schwendener, Reto & Schott, Herbert. (2010), Liposome Formulations of Hydrophobic Drugs, Methods in molecular biology (Clifton, N.J.), 605, 129-38, doi:10.1007/978-1-60327-360-2_8; Olusanya TOB, Haj Ahmad RR, Ibegbu DM, Smith JR, Elkordy AA. Liposomal Drug Delivery Systems and Anticancer Drugs, Molecules 2018, 23(4):907, doi:10.3390/molecules23040907; Bulbake U, Doppalapudi S, Kommineni N, Khan W. Liposomal Formulations in Clinical Use: An Updated Review, Pharmaceutics 2017, 9(2): 12, doi:10.3390/pharmaceutics9020012). Liposomes, spherical vesicles comprising a bilayer of lipids with an internal aqueous cavity, are known to the skilled person and are particularly useful for the administration of the lipophilic 5-FU prodrugs of the invention. The 5-FU prodrugs of the invention can be incorporated into lipid bilayer membranes of liposomes, which may, for example, be administered orally or parenterally.

A preferred embodiment of a compound according to the invention can be prepared, for example, according to the phosphoramidite route or the H-Phosphonate route (Gollnest, T. 2015, Das TriPPPro-Konzept: Entwicklung und Charakterisierung von antiviral en Nukleosidtriphosphat-Prodrugs, Dissertation, Hamburg University, urn:nbn:de:gbv:18-78516; Zhao, C. 2019, Non-Symmetrically-Masked TriPPPro Prodrugs and γ-Modified Nucleoside Triphosphate Compounds as Potential Antivirals against HIV, Disseration, Hamburg University, urn:nbn:de:gbv: 18-95886; Jia, X. 2020 Membrane-Permeable Nucleoside Triphosphate Prodrugs of Anti-HIV Active Nucleoside Analogues: γ-(Phosphate or Phosphonate)-Modified Nucleotide Analogues, Dissertation, Hamburg University, urn:nbn:de:gbv: 18-ediss-86856). An exemplary scheme of the H-Phosphonate route for preferred embodiments of the invention is given below:

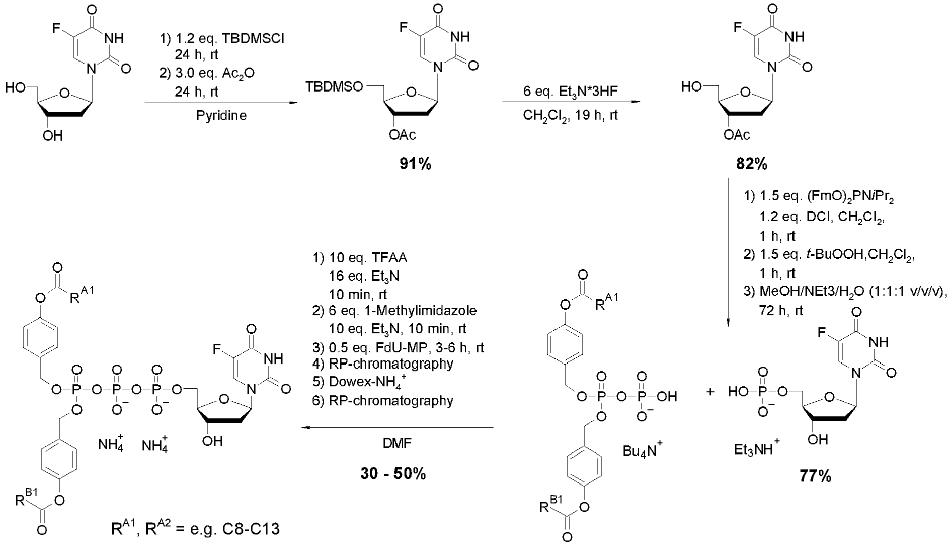

In the scheme above, both masks R are identical and R is C₈₋₁₃ akyl. The synthesis route (also denoted "Variant A" herein) can, however, also be used to synthesize asymmetrically masked 5-Fluorouracil nucleoside triphosphates of the invention. For further details see section "general procedures" below.

An exemplary scheme of the phosphoramidite route (also denoted "variant B" herein), which is particularly useful for the preparation of asymmetrically masked 5-Fluorouracil nucleoside triphosphates, is shown below using the example of the synthesis of two compounds. Residues R^{A1} and R^{B1} are C7 alkyl (R^{A1}) and C9 alkyl (R^{B1}), or C10 (R^{A1}) and C12 alkyl (R^{B1}). For further details see section "general procedures" below. 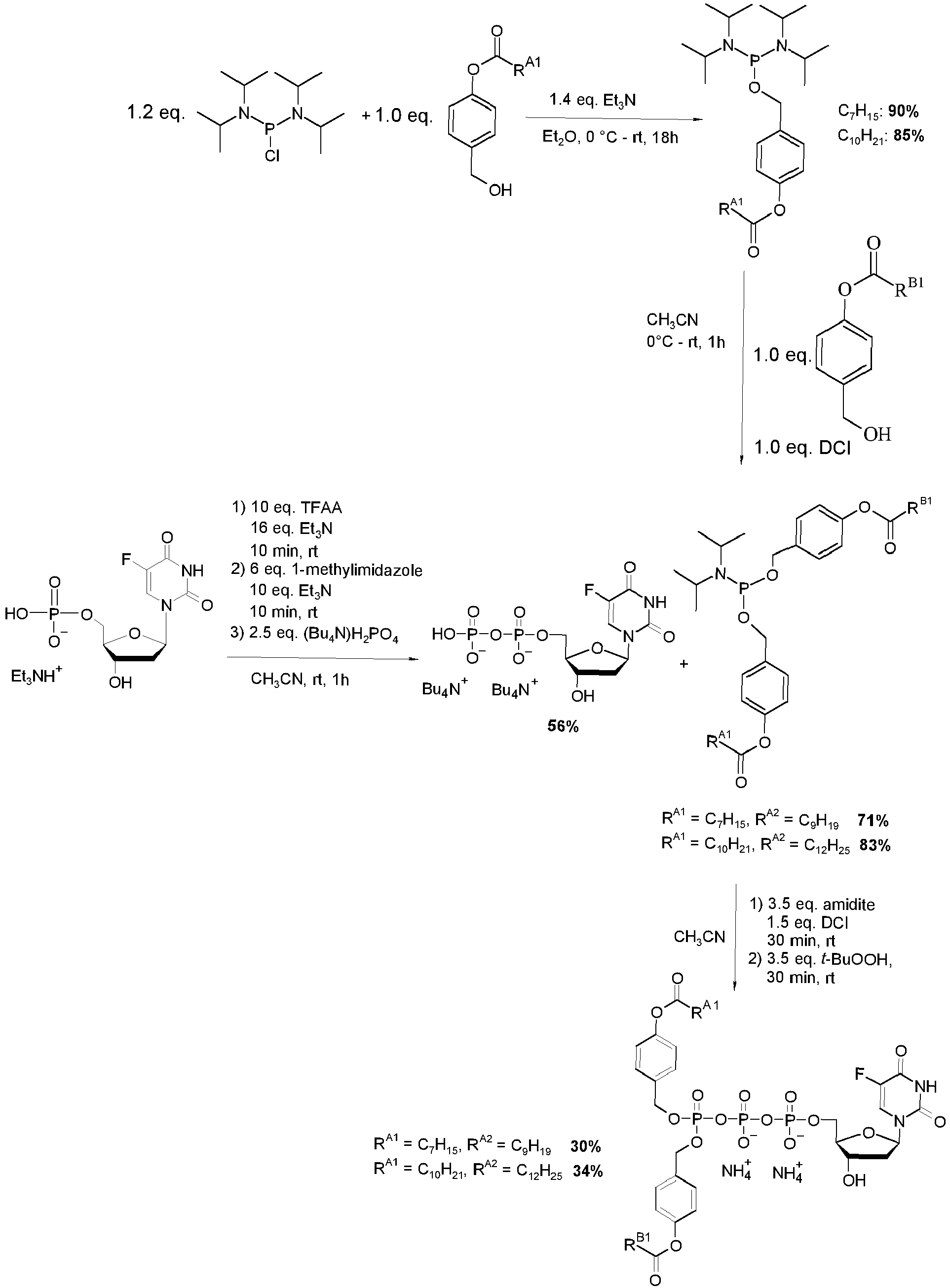 In a still further aspect, the invention also relates to a method of treatment of cancer, the method comprising administering to a patient in need thereof an effective amount of a 5-Fluorouracil nucleoside triphosphate prodrug of the invention. In a preferred embodiment of the method of the invention the 5-Fluorouracil nucleoside triphosphate prodrug of the invention is in the form of a pharmaceutical composition or dosage form according to the invention. In a preferred embodiment of the method of the invention the 5-Fluorouracil nucleoside triphosphate prodrug of the invention is administered in the form of a liposome or liposome formulation.

Preferably, the method is a method for treating a cancer disease selected from colon carcinoma, breast cancer, esophageal cancer, head and neck cancer, stomach cancer, anal cancer and squamous cell carcinoma.

It is also possible to combine the administration of a 5-Fluorouracil nucleoside triphosphate prodrug of the invention with the administration of one or more other drugs. If a combination of drugs, including a 5-FU NTP prodrug of the invention, is administered, it is especially preferred to combine the 5-FU NTP prodrug of the invention with a drug or drugs that have already successfully combined with 5-FU, in particular treatment regimens for treating cancer, e.g. with leucovorin (folinic acid) calcium and irinotecan hydrochloride ("FOLFIRI") or with leucovorin and oxaliplatin ("FOLFOX").

The invention is explained in more detail below with reference to the accompanying figures and exemplary embodiments for illustrative purposes only.

### BRIEF DESCRIPTION OF THE DRAWINGS

FIG. 1 schematically shows the entry into the cell of an embodiment of a 5-Fluorouracil nucleoside triphosphate prodrug of the invention and the release of the corresponding 5-Fluorouracil nucleoside triphosphate after intracellular cleavage of the prodrug. Nu^{⊖}= nucleophile; T_{1/2} = half-life; 5FU-TP = 5-Fluorouracil nucleoside triphosphate. The residues masking the terminal phosphate are denoted with "A" and "B".
FIG. 2. IC₅₀ values of 5-Fluorouracil nucleoside triphosphate prodrugs (JW-1, JW-2, JW-3, JW-4, JW-5, JW6) according to the invention, compared to 5-Fluorouracil (5FU), for a breast cancer cell line (MDA-MB231).
FIG. 3. IC₅₀ values of 5-Fluorouracil nucleoside triphosphate prodrugs (JW-1, JW-2, JW-3, JW-4, JW-5, JW6) according to the invention, compared to 5-Fluorouracil (5FU), for colon cancer cell line HTC116.
FIG. 4. IC₅₀ values of 5-Fluorouracil nucleoside triphosphate prodrugs (JW-1, JW-2, JW-3, JW-4, JW-5, JW6) according to the invention, compared to 5-Fluorouracil (5FU), for colon cancer cell line HT29.
FIG. 5. IC₅₀ values of 5-Fluorouracil nucleoside triphosphate prodrugs (JW-1, JW-2, JW-3, JW-4, JW-5, JW6) according to the invention, compared to 5-Fluorouracil (5FU), for colon cancer cell line SW620.
FIG. 6. Percentage of living (viable) cells of colon cancer cell line SW620 in the presence of different concentrations of selected symmetrically masked 5-Fluorouracil nucleoside triphosphate prodrugs JW-1 and JW-4 according to the invention.
FIG. 7. Percentage of viable cells in the presence of different concentrations of selected asymmetrically masked 5-Fluorouracil nucleoside triphosphate prodrugs (JW-7, JW-8, JW-9) according to the invention for the determination of the corresponding IC₅₀ values, compared to 5-Fluorouracil (5FU), for colon cancer cell line CaCo2.
FIG. 8. Percentage of viable cells in the presence of different concentrations of selected asymmetrically masked 5-Fluorouracil nucleoside triphosphate prodrugs (JW-7, JW-8, JW-9) according to the invention for the determination of the corresponding IC₅₀ values, compared to 5-Fluorouracil (5FU), for colon cancer cell line HT29.

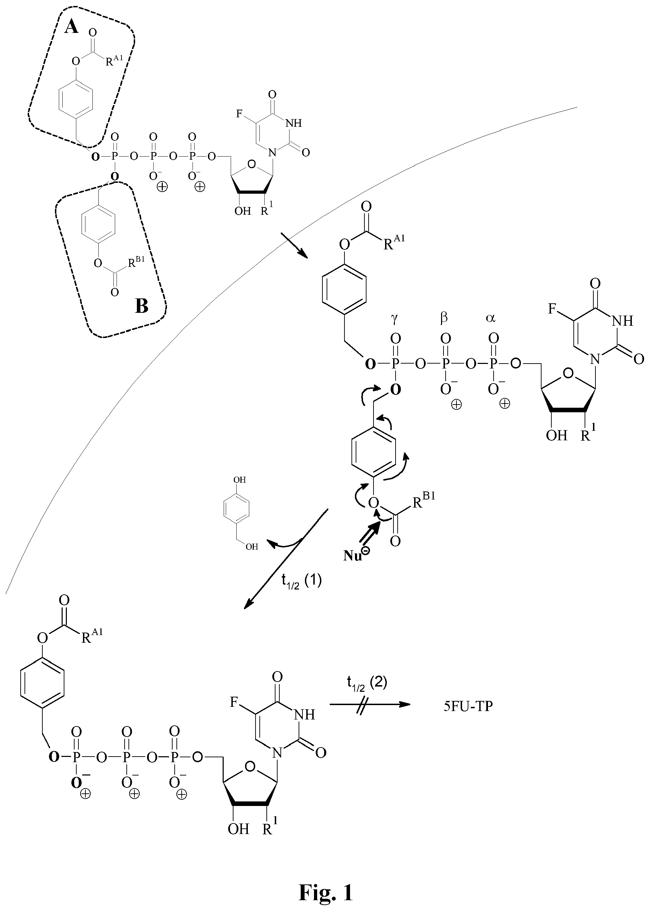

FIG. 1 shows, for an exemplary embodiment of a 5-Fluorouracil nucleoside triphosphate prodrug according to the invention, schematically its introduction as a double-modified prodrug into a cell whose cell membrane is symbolized here by an arcuate line, as well as the assumed course of release of the corresponding 5-Fluorouracil nucleoside triphosphate (FU-TP). The two otherwise negatively charged oxygen atoms (in bold face type) of the hydroxyl groups of the terminal phosphate (γ-phosphorus atom) in the FU-TP prodrug are substituted with two groups (denoted "A" and "B"), which are both acyloxybenzyl residues. The residues masking the γ-phosphate of the nucleoside triphosphate can be identical or different groups. The masks can be cleaved hydrolytically or enzymatically. In the cell, the masks are therefore rapidly removed after an initial nucleophilic attack on the ester group, which may be catalyzed by an esterase in the cell, for example, and subsequent spontaneous degradation. The first mask ("B") is cleaved at a first rate such that the starting prodrug has a first half-life time t_{1/2} (1), the second mask (A) at a second rate, such that the monomodified prodrug, i.e. the prodrug only carrying one of the original masks, has a second half-time time t_{1/2} (2). Finally, 5-Fluorouracil nucleoside triphosphate is released from the prodrug within the cell.

### General procedures

### Procedure 1: synthesis of acyl chlorides

Under dry conditions the carbonic acid (1.0 eq) was dissolved in dry dichloromethane (CH₂Cl₂) and cooled to 0 °C before dropwise addition of oxalyl chloride (1.2 eq, (COCl)₂) and catalytic amounts of *N*,*N*-dimethylformamide (DMF). After reflux for three hours all volatiles were removed under reduced pressure. The obtained crude product was used without further purifications.

### Procedure 2: synthesis of 4-(hydroxymethyl)-alkanoates

Under dry conditions 4-Hydroxybenzyl alcohol (1.0 eq) was dissolved in dry tetrahydrofurane (THF), triethylamine (1.0 eq, Et₃N) and 4-dimethylaminopyridine (0.1 eq., DMAP) was added. After cooling the reaction mixture to 0 °C the acyl chloride (1.2 eq) was added dropwise before stirring the reaction for two hours at room temperature. Then, the reaction mixture was filtrated and all volatiles were removed under reduced pressure. The crude product was purified on silica gel.

### Procedure 3: synthesis of symmetric Bis(4-alkanoyloxybenzyl)-phosphonates

Under dry conditions the 4-(hydroxymethyl)-alkanoate (2.0 - 2.2 eq.) was dissolved in dry pyridine and diphenyl phosphite (1.0 eq.) was added dropwise. After stirring at 40 °C for three hours, all volatiles were removed under reduced pressure. The residue was evaporated twice with toluene and once with dichloromethane before it was recrystallized in methanol.

### Procedure 4: synthesis of 4-alkanoyloxybenzylbis(N,N-diisopropylamino)-phosphor-amidites

Under dry conditions chlorobis(*N*.*N*-di*iso*propylamino)-phosphoramidite (1.2 eq.) was dissolved in dry diethylether (Et₂O) and the 4-(hydroxymethyl)-alkanoate (1.0 eq.) and Et₃N (1.4 eq.) dissolved in Et₂O were added dropwise at 0 °C. After stirring for 16 hours at room temperature the suspensions was filtrated and all volatiles were removed under reduced pressure. The crude product was purified on silica gel.

### Procedure 5: synthesis of asymmetric Bis(4-alkanoyloxybenzyl)-N,N-diisopropylamino-phosphoramidites

Under dry conditions the 4-alkanoyloxybenzylbis(N,N-diisopropylamino)-phosphoramidite (1.5 eq) was dissolved in dry CH₃CN/THF (1:1) and cooled to 0 °C followed by dropwise addition of a mixture of the 4-(hydroxymethyl)alkanoate (1.0 eq.) and DCI-activator (1.0 eq.) in CH₃CN. The reaction was stirred for 18 hours at room temperature before all volatiles were removed under reduced pressure. The resiude was dissolved in petroleum ether/Et₃N (9:1) and filtrated. The crude product was purified on silica gel.

### Procedure 6: synthesis of TriPPPro compounds

### Variant A: H-phosphonate route:

Both the synthesis of the pyrophosphate and the Tri*PPP*ro-compound were carried out under dry conditions. The phosphonate (1.0 eq.) was suspended in dry CH₃CN or THF, heated to 50 °C and *N*-chlorosuccinimide (2.0 eq., NCS) was added. The reaction was stirred for one hour at 50 °C before adding the mixture dropwise to a solution of tetrabutylammonium phosphate monobasic in dry acetonitrile (0.4 mol/L). The solution was stirred for another hour at 50 °C before all volatiles were removed under reduced pressure. The residue was dissolved in 20 mL dichloromethane and washed once with NH₄OAc solution (1 mol/L) and once with water. Phase separation was accelerated with centrifugation. The solvent was removed under reduced pressure and the resulting pyrophosphate was used without further purification (stored under nitrogen and -20 °C).

The pyrophosphate (1.0 eq.) was dissolved in 5 mL dry DMF and cooled to 0 °C. Then a 0 °C precooled solution of triethylamine (10 eq.) and trifluoroacetic anhydride (16 eq., TFAA) in 5 mL dry CH₃CN was added dropwise and stirred for 10 min at 0 °C. All volatiles were removed under reduced pressure and the residue dissolved in 5 mL dry CH₃CN or DMF. Then triethylamine (10 eq.) and 1-methylimidazole (6 eq.) were added and the solution was stirred for 10 min at room temperature. Afterwards the monophosphate dissolved in dry CH₃CN or DMF was added dropwise and the reaction mixture was stirred at room temperature until full conversion was observed by RP-HPLC. All volatiles were removed under reduced pressure and the residue was purified on RP₁₈ silica gel with automated flash chromatography (H₂O/CH₃CN, 100:0 to 0:100 in 45 min, 20 mL/min) followed by ion exchange (NH₄⁺) and further purification on RP₁₈ silica gel with automated flash chromatography (H₂O/CH₃CN, 100:0 to 0:100 in 30 min, 20 mL/min).

### Variant B: Phosphoramidite route:

Under dry conditions, the FdU-diphosphate (1.0 eq.) and the Bis(4-alkanoyloxybenzyl)-*N*,*N-*diisopropylamino-phosphoramidite (3.0 eq.) were coevaporated with dry CH₃CN and dried for three hours under vacuum. Afterwards the residue was dissolved in dry CH₃CN and cooled to 0 °C followed by dropwise addition of DCI-Activator (1.2 eq., 0.25 mol/L in CH₃CN). After stirring for 30 minutes at room temperature *tert*-butylhydroperoxide (3.5 eq., 5.5 mol/L in decane) was added and the reaction mixture was stirred for another 30 minutes. Afterwards all volatiles were removed under reduced pressure and the crude product was purified on RP₁₈ silica gel with automated flash chromatography (H₂O/CH₃CN, 100:0 to 0:100 in 45 min, 20 mL/min) followed by ion exchange (NH₄⁺) and further purification on RP₁₈ silica gel with automated flash chromatography (H₂O/CH₃CN, 100:0 to 0:100 in 30 min, 20 mL/min).

### Compounds:

### 4-(hydroxymethyl)-phenylpentanoate

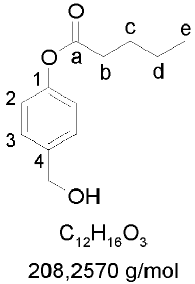

The reaction was carried out according to general procedure 2 with 4.25 g (34.1 mmol, 1.2 eq.) 4-hydroxybenzylalcohol, 3.94 mL (28.4 mmol, 1.0 eq.) Et₃N and 350 mg (2.85 mmol, 0.1 eq.) 4-dimethylaminopyridine (DMAP) in 30 mL dry THF followed by dropwise addition of 4.50 mL (28.4 mmol, 1.0 eq.) pentanoyl chloride in 20 mL dry THF. The crude product was purified on silica gel (petroleum ether/EtOAc 4:1). Yield: 4.75 g (22.8 mmol, 80%) as a colourless oil.

### 4-(hydroxymethyl)-phenyloctanoate

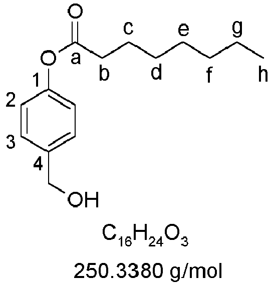

The reaction was carried out according to general procedure 2 with 4.08 g (32.9 mmol, 1.2 eq.) 4-hydroxybenzylalcohol, 3.80 mL (27.4 mmol, 1.0 eq.) Et₃N and 335 mg (2.74 mmol, 0.1 eq.) 4-dimethylaminopyridine (DMAP) in 30 mL dry THF followed by dropwise addition of 4.55 mL (27.4 mmol, 1.0 eq.) octanoyl chloride in 20 mL dry THF. The crude product was purified on silica gel (petroleum ether/EtOAc 4:1). Yield: 4.59 g (18.3 mmol, 67%) as a pale yellow solid.

### 4-(hydroxymethyl)-phenylnonanoate

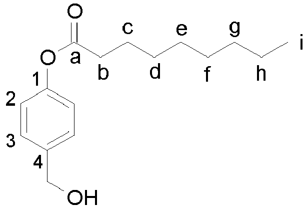

C₁₆H₂₄O₃ 264.3650 g/mol

The reaction was carried out according to general procedure 2 with 4.05 g (32.6 mmol, 1.2 eq.) 4-hydroxybenzylalcohol, 3.77 mL (27.2 mmol, 1.0 eq.) Et₃N and 332 mg (2.72 mmol, 0.1 eq.) 4-dimethylaminopyridine (DMAP) in 40 mL dry THF followed by dropwise addition of 4.90 mL (27.2 mmol, 1.0 eq.) nonanoyl chloride in 20 mL dry THF. The crude product was purified on silica gel (petroleum ether/EtOAc 4:1). Yield: 5.60 g (21.2 mmol, 78%) as a colorless solid.

### 4-(Hydroxymethyl)-phenyldecanoate

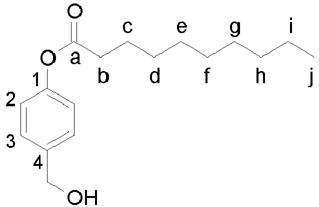

C₁₇H₂₆O₃ 278.3920 g/mol

The reaction was carried out according to general procedure 2 with 3.98 g (32.1 mmol, 1.2 eq.) 4-hydroxybenzyl alcohol and 3.70 mL (26.7 mmol, 1.0 eq.) Et₃N in 40 mL dry THF followed by dropwise addition of 5.20 mL (26.7 mmol, 1.0 eq.) decanoyl chloride in 10 mL dry THF. The crude product was purified on silica gel (petroleum ether/EtOAc 4:1). Yield: 5.76 g (20.7 mmol, 77%) as a colorless solid.

### 4-(Hydroxymethyl)-phenylundecanoate

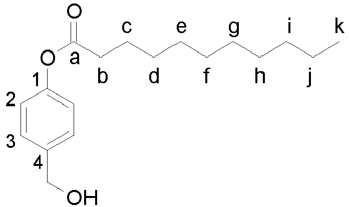

C₁₈H₂₈O₃ 292.4190 g/mol

The undecanoyl chloride was prepared according to general procedure 1 with 5.35 g (28.7 mmol, 1.0 eq.) undecanoic acid, 2.96 mL (34.5 mmol, 1.2 eq.) COCl₂ and 2 drops of dry DMF in 40 mL dry CH₂Cl₂. The esterification was carried out according to general procedure 2 with 4.22 g (34.5 mmol, 1.2 eq.) 4-hydroxybenzyl alcohol, 3.98 mL (28.7 mmol, 1.0 eq.) Et₃N and 348 mg (2.87 mmol, 0.1 eq.) 4-Dimethylaminopyridine (DMAP) in 40 mL dry THF followed by dropwise addition of the freshly prepared acyl chloride in 20 mL dry THF. The crude product was purified on silica gel (petroleum ether/EtOAc 4:1). Yield: 7.07 g (24.2 mmol, 84%) as a colorless solid.

### 4-(hydroxymethyl)-phenyldodecanoate

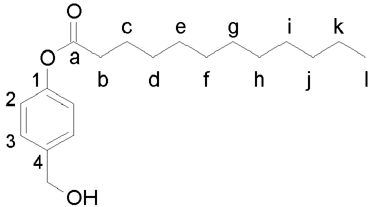

C₁₉H₃₀O₃ 306.4460 g/mol

The reaction was carried out according to general procedure 2 with 4.07 g (32.8 mmol, 1.2 eq.) 4-hydroxybenzyl alcohol, 3.79 mL (27.3 mmol, 1.0 eq.) Et₃N and 334 mg (2.73 mmol, 0.1 eq.) 4-dimethylaminopyridine (DMAP) in 40 mL dry THF followed by dropwise addition of 6.50 mL (27.3 mmol, 1.0 eq.) dodecanoyl chloride in 10 mL dry THF. The crude product was purified on silica gel (petroleum ether/EtOAc 3:1). Yield: 6.93 g (22.6 mmol, 83%) as a colorless solid.

### 4-(hydroxymethyl)-phenyltridecanoate

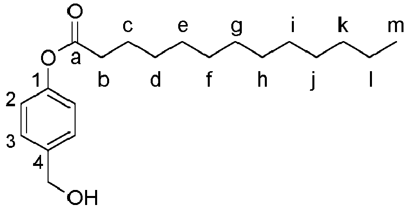

C₂₀H₃₂O₃ 320.4730 g/mol

The tridecanoyl chloride was prepared according to general procedure 1 with 10.0 g (46.8 mmol, 1.0 eq.) tridecanoic acid, 4.81 mL (56.1 mmol, 1.2 eq.) COCl₂ and 2 drops of dry DMF in 40 mL dry CH₂Cl₂. The esterification was carried out according to general procedure 2 with 6.96 g (56.1 mmol, 1.2 eq.) 4-hydroxybenzyl alcohol, 7.78 mL (56.1 mmol, 1.0 eq.) Et₃N and 571 mg (4.68 mmol, 0.1 eq.) 4-dimethylaminopyridine (DMAP) in 40 mL dry THF followed by dropwise addition of the freshly prepared acyl chloride in 40 mL dry THF. The crude product was purified on silica gel (petroleum ether/EtOAc 4:1). Yield: 11.3 g (35.3 mmol, 75%) as a colorless solid.

### 4-(hydroxymethyl)-phenyltetradecanoate

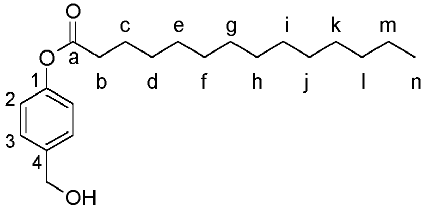

C₂₁H₃₄O₃ 334.5000 g/mol

The reaction was carried out according to general procedure 2 with 3.99 g (32.2 mmol, 1.2 eq.) 4-hydroxybenzyl alcohol, 3.72 mL (26.9 mmol, 1.0 eq.) Et₃N and 327 mg (2.69 mmol, 0.1 eq.) 4-dimethylaminopyridine (DMAP) in 30 mL dry THF followed by dropwise addition of 7.28 mL (26.9 mmol, 1.0 eq.) tetradecanoyl chloride in 10 mL dry THF. The crude product was purified on silica gel (petroleum ether/EtOAc 4:1). Yield: 5.75 g (17.2 mmol, 64%) as a colorless solid.

### 4-(hydroxymethyl)-phenyloctadecanoate

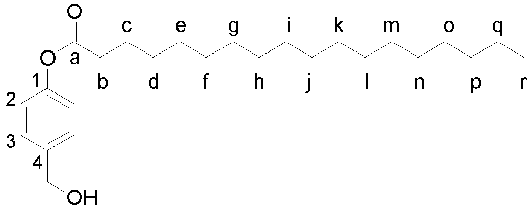

C₂₅H₄₂O₃ 390,6080 g/mol

The reaction was carried out according to general procedure 2 with 2.21 g (17.8 mmol, 1.2 eq.) 4-hydroxybenzyl alcohol, 2.05 mL (14.8 mmol, 1.0 eq.) Et₃N and 182 mg (1.48 mmol, 0.1 eq.) 4-dimethylaminopyridine (DMAP) in 30 mL dry THF followed by dropwise addition of 5.00 mL (14.8 mmol, 1.0 eq.) octadecanoyl chloride in 10 mL dry THF. The crude product was purified on silica gel (petroleum ether/EtOAc 5:1). Yield: 4.75 g (12.2 mmol, 82%) as a colorless solid.

### Bis(4-nonanoyloxybenzyl)-phosphonate

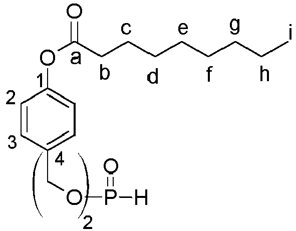

C₃₂H₄₇O₄P 574.7011 g/mol

The reaction was carried out according to general procedure 3 with 1.76 g (6.64 mmol, 2.0 equiv) 4-(hydroxymethl)-phenylnonanoate and 0.64 mL (3.32 mmol, 1.0 equiv) diphenyl phosphite in 10 ml dry pyridine. Yield: 0.67 g (1.17 mmol, 35%) as a colourless solid.

### Bis(4-decanoyloxybenzyl)-phosphonate

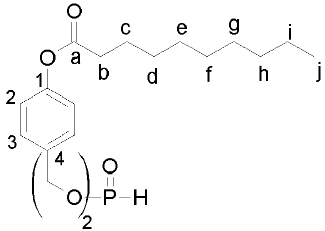

C₃₄H₅₁O₇P 602.7554 g/mol

The reaction was carried out according to general procedure 3 with 1.30 g (4.69 mmol, 2.0 equiv) 4-(hydroxymethl)-phenyldecanoate and 0.45 mL (2.34 mmol, 1.0 equiv) diphenyl phosphite in 10 ml dry pyridine. Yield: 821 mg (1.36 mmol, 58%) as a colourless solid.

### Bis(4-undecanoyloxybenzyl)-phosphonate

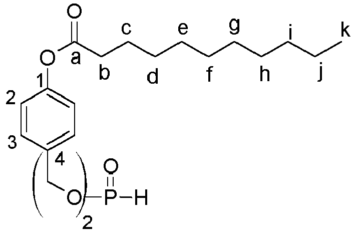

C₃₆H₅₅O₇P 630.8023 g/mol

The reaction was carried out according to general procedure 3 with 2.33 g (7.97 mmol, 2.2 equiv) 4-(hydroxymethl)-phenylundecanoate and 0.70 mL (3.62 mol, 1.0 equiv) diphenyl phosphite in 20 ml dry pyridine. Yield: 1.58 g (2.50 mmol, 69%) as a colourless solid.

### Bis(4-dodecanoyloxybenzyl)-phosphonate

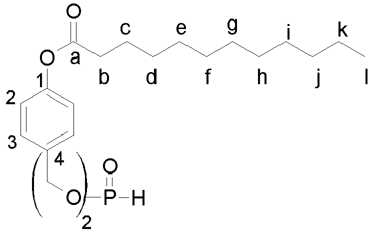

C₃₆H₅₅O₇P 658.8568 g/mol

The reaction was carried out according to general procedure 3 with 3.00 g (9.79 mmol, 2.2 equiv) 4-(hydroxymethl)-phenyldodecanoate and 0.85 mL (4.45 mmol, 1.0 equiv) diphenyl phosphite in 20 ml dry pyridine. Yield: 2.30 g (3.50 mmol, 79%) as a colourless solid.

### Bis(4-tridecanoyloxybenzyl)-phosphonate

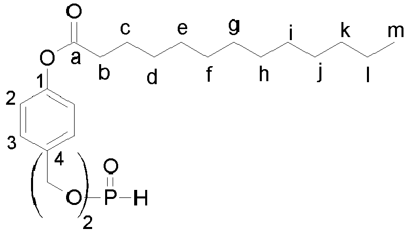

C₄₀H₆₃O₇P 686.9108 g/mol

The reaction was carried out according to general procedure 3 with 3.10 g (10.6 mmol, 2.2 equiv) 4-(hydroxymethl)-phenyltridecanoate and 0.93 mL (4.45 mmol, 1.0 equiv) diphenyl phosphite in 30 ml dry pyridine. Yield: 2.75 g (4.00 mmol, 83%) as a colourless solid.

### Bis(4-tetradecanoyloxybenzyl)-phosphonate

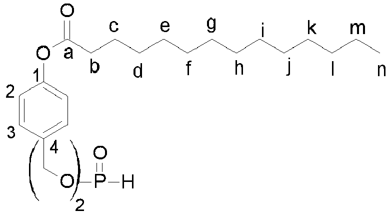

C₄₀H₆₃O₇P 714.9648 g/mol

The reaction was carried out according to general procedure 3 with 0.94 g (2.81 mmol, 2.2 equiv) 4-(hydroxymethl)-phenyltetradecanoate and 0.25 mL (1.28 mmol, 1.0 equiv) diphenyl phosphite in 15 ml dry pyridine. Yield: 771 mg (1.08 mmol, 84%) as a colourless solid.

### (4-pentanoyloxybenzyl)-(4-octadecanoyloxybenzyl)-phosphonate

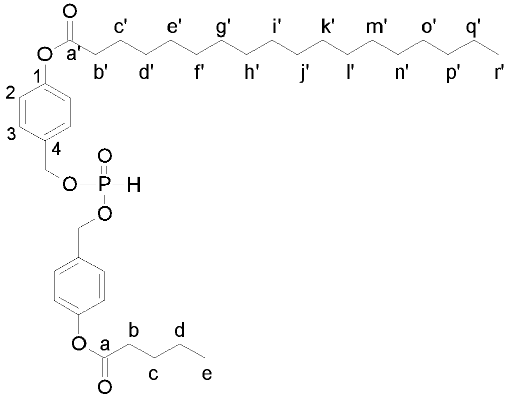

C₃₇H₅₇O₇P 644,8298 g/mol

Under dry conditions 0.28 mL (1.46 mmol, 1.0 eq.) DPP was dissolved in 10 mL dry pyridine and cooled to 0 °C. Then 572 mg (1.46 mmol, 1.0 eq.) 4-(hydroxymethl)-phenyloctadecanoate was added and the suspension was stirred for ten minutes at 0 °C. After stirring for 30 minutes at room temperature 305 mg (1.46 mmol, 1.0 eq.) 4-(hydroxymethl)-phenylpentanoate was added and the reaction mixture was stirred for three hours at room temperature. Afterwards all volatiles were removed under reduced pressure and the residue was coevaporated once with toluene and once with CH₂Cl₂. The crude product was purified on silica gel (petroleum ether/EtOAc 4:1 to 3:1 + 2% AcOH). Yield: 447 mg (693 mmol, 48%) as a colourless solid.

### (Diisoproylamino)dichlorophosphine

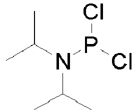

C₆H₁₄Cl₂NP 202.0588 g/mol

Under dry conditions 5.00 mL (57.0 mmol, 1.0 eq.) freshly distilled phosphorus trichloride was dissolved in 40 mL diethyl ether and cooled to 0 °C. Then, a solution of 17 mL (0.12 mol, 2.1 eq) *N*,*N*-di*iso*propylamine in 20 mL diethyl ether was added dropwise. After two hours the reaction mixture was filtrated under dry conditions and the filtrate was concentrated to dryness under reduced pressure. The product was used without further purifications. Yield: 7.91 g (39.1 mmol, 65%) as a yellow oil.

### 4-octanoyloxybenzylbis(N,N-diisopropylamino)-phosphoramidite

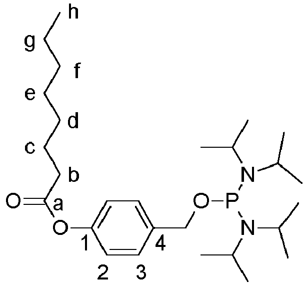

C₂₇H₄₉N₂O₃P 480,6738 g/mol

The reaction was carried out according to general procedure 4 with 520 mg (1.97 mmol, 1.0 eq.) 4-(hydroxymethyl)-phenyloctanoate in 20 mL abs Et₂O and 630 mg (2.36 mmol, 1.2 eq.) chlorobis(*N*.*N*-di*iso*-propylamino)-phosphoramidite and 0.38 mL (2.75 mmol, 1.4 eq.) Et₃N in 10 mL dry Et₂O. The crude product was purified on silica gel (petroleum ether/Et₃N 9:1). Yield: 851 mg (1.77 mmol, 90%) as a colourless oil.

### 4-undecanoyloxybenzylbis(N,N-diisopropylamino)-phosphoramidite

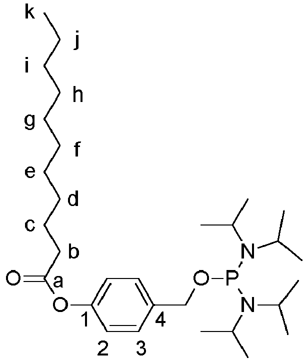

C₃₀H₅₅N₂O₃P 522,7548 g/mol

The reaction was carried out according to general procedure 4 with 550 mg (1.88 mmol, 1.0 eq.) 4-(hydroxymethyl)-phenylundecanoate in 20 mL abs Et₂O and 602 mg (2.26 mmol, 1.2 eq.) chlorobis(*N*.*N*-di*iso*-propylamino)-phosphoramidite and 0.37 mL (2.63 mmol, 1.4 eq.) Et₃N in 10 mL dry Et₂O. The crude product was purified on silica gel (petroleum ether/Et₃N 9:1). Yield: 883 mg (1.60 mmol, 85%) as a colourless oil.

### (4-octanoyloxybenzyl)-(4-decanoyloxybenzyl)-N,N-diisopropylaminophosphoramidite

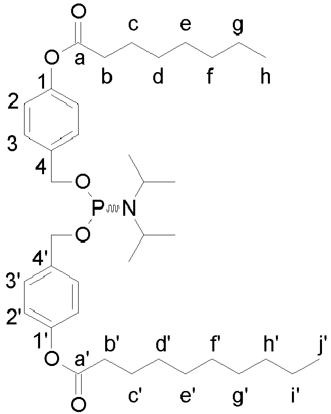

C₃₈H₆₀NO₆P 657,8728 g/mol

The reaction was carried out according to general procedure 5 with 670 mg (1.40 mmol, 1.5 eq.) 4-octanoyloxybenzylbis(N,N-diisopropylamino)-phosphoramidite in 10 mL dry CH₃CN/THF (1:1) and 261 mg (0.93 mmol, 1.0 eq.) 4-(hydroxymethyl)-phenyldecanoate and 3.72 mL DCI-activator (0.93 mmol, 0.25 mol/L in CH₃CN, 1.0 eq.) in 2 mL dry CH₃CN. The crude product was purified on silica gel (petroleum ether/Et₃N 9:1). Yield: 424 mg (661 mmol, 71%) as colourless oil.

### (4-undecanoyloxybenzyl)-(4-tridecanoyloxybenzyl)-N,N-diisopropylaminophosphor-amidite

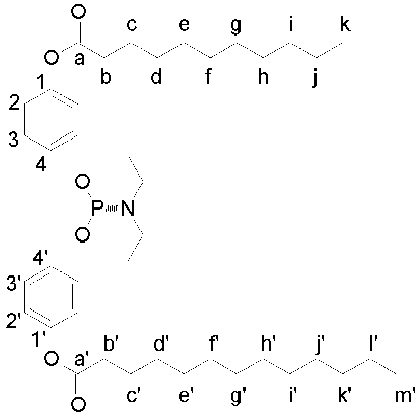

C₄₄H₇₂NO₆P 742,0348 g/mol

The reaction was carried out according to general procedure 5 with 734 mg (1.40 mmol, 1.5 eq.) 4-octanoyloxybenzylbis(N,N-diisopropylamino)-phosphoramidite in 10 mL dry CH₃CN/THF (1:1) and 302 mg (0.94 mmol, 1.0 eq.) 4-(hydroxymethyl)-phenyltridecanoate and 3.74 mL DCI-activator (0.94 mmol, 0.25 mol/L in CH₃CN, 1.0 eq.) in 5 mL dry CH₃CN. The crude product was purified on silica gel (petroleum ether/Et₃N 9:1). Yield: 565 mg (778 mmol, 83%) as colourless oil.

### Bis-O-(9H-fluorene-9-ylmethyl)-N,N-diisopropylaminophosphoramidite

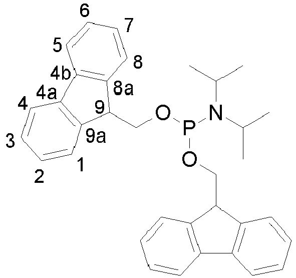

C₃₄H₃₆NO₂P 521.6408 g/mol

The reaction was carried out under dry conditions. 4.00 g (19.8 mmol, 1.0 eq.) (Di*iso*proyl-amino)dichlorophosphine was dissolved in 40 mL THF and cooled to 0 °C. Afterwards, 5.76 mL (4,21 g, 2.1 eq.) Et₃N was added followed by dropwise addition of 7.77 g (39.6 mmol, 2.0 eq.) 9-Fluorenemethanol dissolved in 40 mL THF. The accruing suspension was then stirred for 21 hours before filtration. Ethyl acetate was then added to the filtrate and the organic layer was washed once with PBS-buffer. The aqueous phase was then extracted once with ethyl acetate and the combined organic layers were dried over Na₂SO₄ before removing the solvent under reduced pressure. The crude product was then purified with automated flash column chromatography on silica gel (petroleum ether/EtOAc 96:4, 1% Et₃N). Yield: 8.36 g (16.0 mmol, 81%) as a yellow resin.

### 5-Fluor-3'-O-acetyl-5'-O-tert-butyldimethylsilyl-2'-deoxyuridine

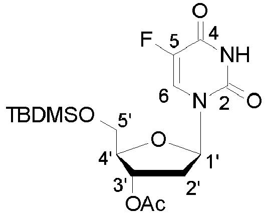

C₁₇H₂₇FN₂O₆Si 402.4944 g/mol

Under dry conditions 3.00 g (8.47 mmol, 1.0 eq.) 5-fluorodeoxyuridine (FdU) was dissolved in 30 mL dry pyridine and 1.53 g (16.9 mmol, 1.2 eq.) *tert*-butyldimethylsilyl chloride (TBDMSCl) was added and the solution was stirred for 18 h at room temperature. 2.40 mL (25.4 mmol, 3.0 eq.) acetic anhydride was added and stirred at room temperature for 18 hours. Afterwards, all volatiles were removed under reduced pressure. The residue was dissolved in CH₂Cl₂ and washed twice with sat. aqueous NaHCO₃ solution and twice with water. After drying the organic layer over Na₂SO₄, the solvent was removed under reduced pressure. The resulting residue was purified on silica gel (petroleum ether/EtOAc 2:1 to 1:1). Yield: 3.12 g (7.75 mmol, 91%) as a colourless solid.

### 5-Fluor-3'-O-acetyl-2'-deoxyuridine

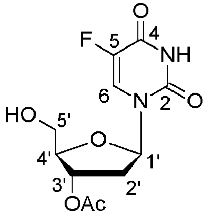

C₁₁H₁₃FN₂O₆ 288.2314 g/mol

Under dry conditions 3.49 g (8.67 mmol, 1.0 eq.) 5-Fluor-3'-*O*-acetyl-5'-*O*-*tert*-butyldimethylsilyl-2'-deoxyuridine was dissolved in 40 mL dry CH₂Cl₂ and 8.47 mL (52.0 mmol, 6.0 eq.) triethylamine trihydrofluoride was added dropwise. After stirring at room temperature for 22 hours, silica gel was added to the reaction mixture. The solvent was removed under reduced pressure and the crude product was purified on silica gel (CH₂Cl₂/MeOH 19:1). Yield: 2.47 g (8.55 mmol, 99%) as a colourless solid.

### FdU-5'-monophosphate

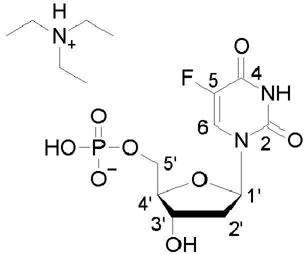

C₉H₁₁FN₂O₈P⁻325.1657

Under dry conditions 1.11 g (3.85 mmol, 1.0 eq.) 5-Fluor-3'-*O*-acetyl-2'-deoxyuridine and 3.01 g (5.77 mmol, 1.5 eq.) Bis-*O*-(9*H*-fluorene-9-ylmethyl)-*N,N*-dii*so*propylaminophosphor-amidite were dissolved in 50 mL dry THF and 18.5 mL (4.61 mmol, 1.2 eq.) DCI-Activator (0.25 mol/L in CH₃CN) was added dropwise at room temperature. After stirring for one hour the reaction mixture was cooled to 0 °C and the oxidation was carried out with dropwise addition of 1.05 mL (5.77 mmol, 1.5 eq.) *tert*-butylhydroperoxide (5.5 mol/L in decane). Afterwards, all volatiles were removed under reduced pressure and the residue was purified on silica gel with automated flash chromatography (CH₂Cl₂/MeOH, 100:0 to 90:10, 40 mL/min) obtaining a colourless foam. For the deprotection the monophosphate was dissolved in 30 mL MeOH/Et₃N/H₂O (1:1:1) and stirred at room temperature for 72 hours. Then, the suspension was filtrated before Et₃N and MeOH were removed under reduced pressure. In the end, the resulting crude product in water was purified on RP₁₈-silica gel with automated flash chromatography (H₂O/CH₃CN, 100:0 to 0:100, 40 mL/min). Yield: 1.27 g (2.97 mmol, 77%) as a colourless resin.

### FdU-5'-diphosphate

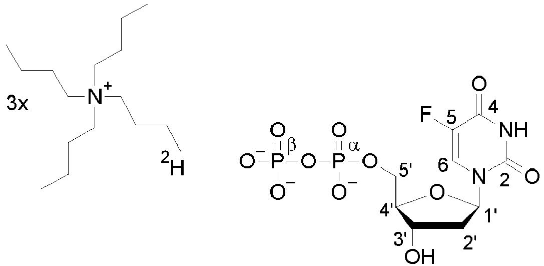

C₉H₁₂FN₂O₁₁P₂⁻405.1445 g/mol

Under dry conditions 195 mg (408 µmol, 1.0 eq.) FdU-5'-monophosphate with 0.90 mL (6.53 mmol, 16 eq.) Et₃N was suspended in 5 mL dry CH₃CN and 0.57 mL (4.08 mmol, 10 eq.) TFAA was added dropwise at room temperature. After stirring for ten minutes, all volatiles were removed under reduced pressure, the residue was dissolved in 5 mL dry CH₃CN and 0.57 mL (4.08 mmol, 10 eq.) Et₃N and 0.19 mL (2.45 mmol, 6.0 eq.) 1-methylimidazole were added. After stirring for ten minutes at room temperature the reaction mixture was added dropwise to 3.05 mL (1.22 mmol, 3.0 eq. 0.40 mol/L) of a solution of tetrabutylammonium phosphate monobasic in dry acetonitrile. Afterwards the reaction was stirred for one hour at room temperature before 5 mL NH₄OAc solution (1 mol/L) was added to the reaction. The resulting mixture was washed once with CHCl₃ before all volatiles were removed under reduced pressure. The residue was purified on RP₁₈ silica gel with automated flash chromatography (H₂O/CH₃CN, 100:0 for 10 min, 100:0 to 0:100 in 20 min, 20 mL/min) followed by ion exchange (Bu₄N⁺) and further purification on RP₁₈ silica gel with automated flash chromatography (H₂O/CH₃CN, 100:0 to 0:100 in 45 min, 20 mL/min).

### C8-TriPPPro (JW-1)

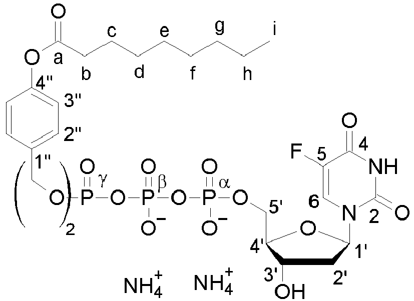

C₂₅H₃₃FN₂O₁₅P₃²⁻713.4588 g/mol

The reaction was carried out according to general procedure 6 (variant A). 320 mg (557 µmol, 1.0 eq.) Bis(4-nonanoyloxybenzyl)-phosphonate in 20 mL dry CH₃CN and 149 mg (1.11 mmol, 2.0 eq.) NCS and 3.48 mL (1.39 mmol, 2.5 eq.) tetrabutylammonium phosphate monobasic in dry acetonitrile (0.4 mol/L) were used. The resulting pyrophosphate (436 mg, 478 µmol, 86% as a yellow resin) was used without further purification.

For the activation, 0.65 mL (4.67 mmol, 10 eq.) TFAA and 1.03 mL Et₃N (7.47 mmol, 16 eq.) in 5 mL dry CH₃CN were used, followed by 0.65 mL (4.67 mmol, 10 eq.) Et₃N and 0.22 mL (2.80 mmol, 6.0 eq.) 1-methylimidazole in 5 mL dry DMF. Afterwards, 99.7 mg (233 µmol, 0.5 eq.) of FdU-5'-monophosphate in 5 mL dry DMF was added and the solution was stirred for 3 h. The crude product was then purified according to the general procedure 4. Yield: 114 mg (113 µmol, 48%) as a colourless cotton.

### C9-TriPPPro (JW-2)

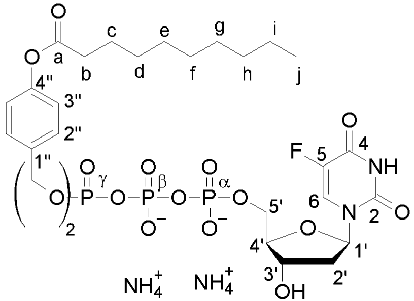

C₂₆H₃₅FN₂O₁₅P₃²⁻727.4858 g/mol

The reaction was carried out according to general procedure 6 (variant A). 200 mg (337 µmol, 1.0 eq.) Bis(4-decanoyloxybenzyl)-phosphonate in 20 mL dry CH₃CN and 90.0 mg (674 µmol, 2.0 eq.) NCS and 2.11 mL (843 µmol, 2.5 eq.) tetrabutylammonium phosphate monobasic in dry acetonitrile (0.4 mol/L) were used. The resulting pyrophosphate (292 mg, 311 µmol, 92% as a yellow resin) was used without further purification.

For the activation 0.43 mL (3.11 mmol, 10 eq.) TFAA and 0.69 mL Et₃N (4.97 mmol, 16 eq.) in 5 mL dry CH₃CN were used, followed by 0.43 mL (3.11 mmol, 10 eq.) Et₃N and 0.15 mL (1.86 mmol, 6.0 eq.) 1-methylimidazole in 5 mL dry DMF. Afterwards, 92.9 mg (217 µmol, 0.7 eq.) of FdU-5'-monophosphate in 5 mL dry DMF was added and the solution was stirred for 3 h. The crude product was then purified according to the general procedure 4. Yield: 75.1 mg (72.1 µmol, 33%) as a colourless cotton.

### C10-TriPPPro (JW-3)

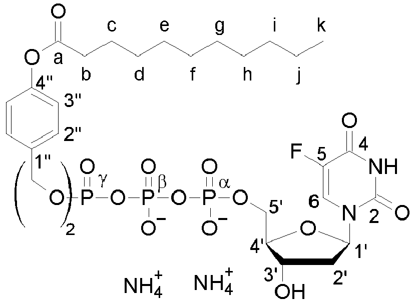

C₂₇H₃₇FN₂O₁₅P₃²⁻741.5128 g/mol

The reaction was carried out according to general procedure 6 (variant A). 215 mg (337 µmol, 1.0 eq.) Bis(4-undecanoyloxybenzyl)-phosphonate in 20 mL dry CH₃CN and 90.0 mg (674 µmol, 2.0 eq.) NCS and 2.11 mL (843 µmol, 2.5 eq.) tetrabutylammonium phosphate monobasic in dry acetonitrile (0.4 mol/L) were used. The resulting pyrophosphate (300 mg, 319 µmol, 92% as a yellow resin) was used without further purification.

For the activation, 0.44 mL (3.19 mmol, 10 eq.) TFAA and 0.71 mL Et₃N (5.10 mmol, 16 eq.) in 5 mL dry CH₃CN were used, followed by 0.44 mL (3.19 mmol, 10 eq.) Et₃N and 0.15 mL (1.91 mmol, 6.0 eq.) 1-methylimidazole in 5 mL dry DMF. Afterwards, 95.3 mg (223 µmol, 0.7 eq.) of FdU-5'-monophosphate in 5 mL dry DMF was added and the solution was stirred for 4 h. The crude product was then purified according to the general procedure 4. Yield: 55.6 mg (52.0 µmol, 23%) as a colourless cotton.

### C11-TriPPPro (JW-4)

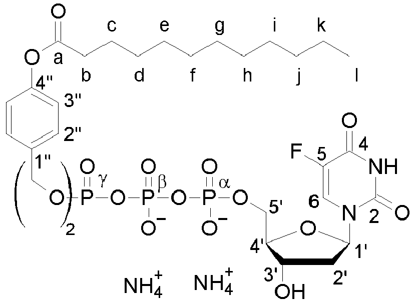

C₂₈H₃₉FN₂O₁₅P₃²⁻755.5398 g/mol

The reaction was carried out according to general procedure 6 (variant A). 204 mg (337 µmol, 1.0 eq.) Bis(4-dodecanoyloxybenzyl)-phosphonate in 20 mL dry CH₃CN and 90.5 mg (647 µmol, 2.0 eq.) NCS and 2.11 mL (843 µmol, 2.5 eq.) tetrabutylammonium phosphate monobasic in dry acetonitrile (0.4 mol/L) were used. 241 mg (242 µmol) of the resulting pyrophosphate (268 mg, 269 µmol, 80% as a yellow resin) was used without further purification.

For the activation 0.34 mL (2.42 mmol, 10 eq.) TFAA and 0.54 mL Et₃N (3.87 mmol, 16 eq.) in 5 mL dry CH₃CN were used, followed by 0.34 mL (2.42 mmol, 10 eq.) Et₃N and 0.11 mL (1.45 mmol, 6.0 eq.) 1-methylimidazole in 5 mL dry DMF. Afterwards, 72.4 mg (169 µmol, 0.7 eq.) of FdU-5'-monophosphate in 5 mL dry DMF was added and the solution was stirred for 5 h. The crude product was then purified according to the general procedure 4. Yield: 55.4 mg (50.5 µmol, 30%) as a colourless cotton.

### C12-TriPPPro (JW-5)

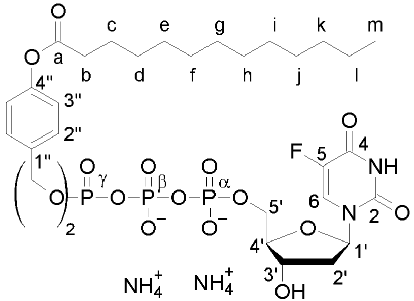

C₂₉H₄₁FN₂O₁₅P₃²⁻769.5668 g/mol

The reaction was carried out according to general procedure 6 (variant A). 254 mg (386 µmol, 1.0 eq.) Bis(4-tridecanoyloxybenzyl)-phosphonate in 20 mL dry CH₃CN and 103 mg (771 µmol, 2.0 eq.) NCS and 2.41 mL (964 µmol, 2.5 eq.) tetrabutylammonium phosphate monobasic in dry acetonitrile (0.4 mol/L) were used. The resulting pyrophosphate (247 mg, 241 µmol, 63% as a yellow resin) was used without further purification.

For the activation 0.34 mL (2.48 mmol, 10 eq.) TFAA and 0.55 mL Et₃N (3.97 mmol, 16 eq.) in 5 mL dry CH₃CN were used, followed by 0.34 mL (2.48 mmol, 10 eq.) Et₃N and 0.12 mL (1.49 mmol, 6.0 eq.) 1-methylimidazole in 5 mL dry DMF. Afterwards, 74.2 mg (174 µmol, 0.7 eq.) of FdU-5'-monophosphate in 5 mL dry DMF was added and the solution was stirred for 5 h. The crude product was then purified according to the general procedure 4. Yield: 61.3 mg (54.5 µmol, 31%) as a colourless cotton.

### C13-TriPPPro (JW-6)

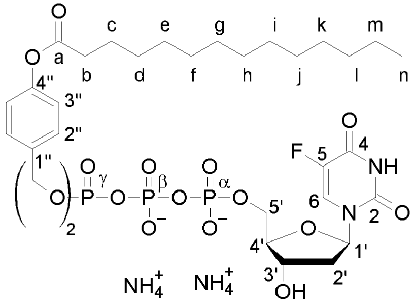

C₃₀H₄₃FN₂O₁₅P₃²⁻783.5938 g/mol

The reaction was carried out according to general procedure 6 (variant A). 410 mg (573 µmol, 1.0 eq.) Bis(4-tetradecanoyloxybenzyl)-phosphonate in 20 mL dry CH₃CN and 153 mg (1.15 mmol, 2.0 eq.) NCS and 3.58 mL (1.43 mmol, 2.5 eq.) tetrabutylammonium phosphate monobasic in dry acetonitrile (0.4 mol/L) were used. 233 mg (227 µmol) of the resulting pyrophosphate (508 mg, 483 µmol, 84% as a yellow resin) was used without further purification.

For the activation, 0.32 mL (2.28 mmol, 10 eq.) TFAA and 0.50 mL Et₃N (3.64 mmol, 16 eq.) in 5 mL dry CH₃CN were used, followed by 0.32 mL (2.28 mmol, 10 eq.) Et₃N and 0.11 mL (1.37 mmol, 6.0 eq.) 1-methylimidazole in 5 mL dry DMF. Afterwards, 68.0 mg (159 µmol, 0.7 eq.) of FdU-5'-monophosphate in 5 mL dry DMF was added and the solution was stirred for 5 h. The crude product was then purified according to the general procedure 4. Yield: 23.5 mg (20.4 µmol, 13%) as a colourless cotton

### C4/C17-TriPPPro (JW-7)

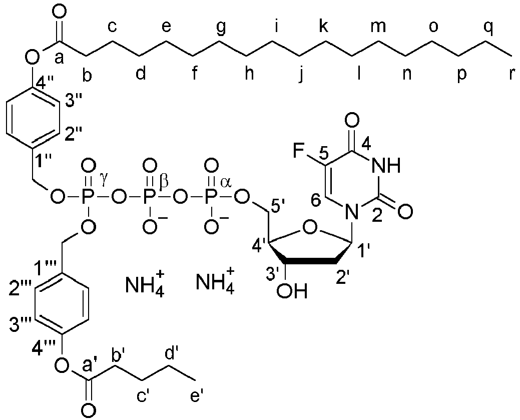

C₄₆H₆₆FN₂O₁₈P₃²⁻1046.9508 g/mol

The reaction was carried out according to general procedure 4 (variant A). 295 mg (468 µmol, 1.0 eq.) (4-octadecanoyloxybenzyl)-(4-pentanoyloxybenzyl)-phosphonate in 20 mL dry CH₃CN and 126 mg (935 µmol, 2.0 eq.) NCS and 2.92 mL (1.17 mmol, 2.5 eq.) tetrabutylammonium phosphate monobasic in dry acetonitrile (0.4 mol/L) were used. The resulting pyrophosphate (307 mg, 312 µmol, 67% as a yellow resin) was used without further purification.

For the activation, 0.43 mL (3.08 mmol, 10 eq.) TFAA and 0.68 mL Et₃N (4.93 mmol, 16 eq.) in 5 mL dry CH₃CN were used, followed by 0.43 mL (3.08 mmol, 10 eq.) Et₃N and 0.15 mL (1.85 mmol, 6.0 eq.) 1-methylimidazole in 5 mL dry DMF. Afterwards, 73.1 mg (154 µmol, 0.5 eq.) of FdU-5'-monophosphate in 5 mL dry DMF was added and the solution was stirred for 3 h. The crude product was then purified according to the general procedure 4. Yield: 31.3 mg (28.6 µmol, 19%) as a colourless cotton.

### C7/C9-TriPPPro (JW-8)

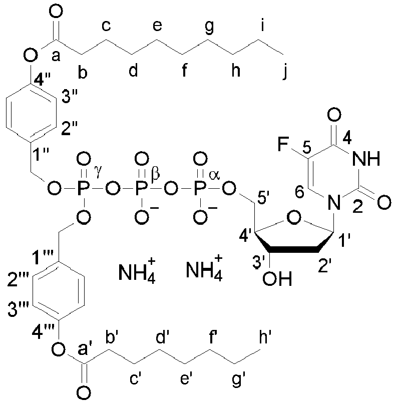

C₄₁H₅₆FN₂O₁₈P₃²⁻976.8158 g/mol

The reaction was carried out according to general procedure 6 (variant B). 102 mg (88.0 µmol, 1.0 eq.) FdU-diphosphate, 182 mg (265 µmol, 3.0 eq.) (4-pentanoyloxybenzyl)-(4-decanoyl-oxybenzyl)-*N,N*-di*iso*propylaminophosphoramidite and 0.53 mL (133 µmol, 0.25 mol/L, 1-2 eq.) DCI-activator were used followed by oxidation with 0.06 mL (310 µmol, 5.5 m/L in decane, 3.5 eq.) tert-butylhydroperoxide. The crude product was then purified according to the general procedure 6 (variant B). Vield: 27.1 mg (26.7 µmol, 30%) as a colourless cotton.

### C10/C12-TriPPPro (JW-9)

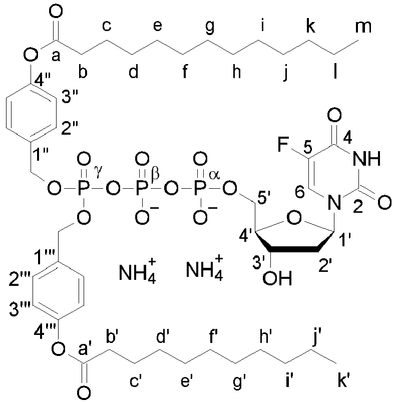

C₄₇H₆₈FN₂O₁₈P₃²⁻ 1060.9778 g/mol

The reaction was carried out according to general procedure 6 (variant B). 55.0 mg (49.4 µmol, 1.0 eq.) FdU-diphosphate, 102 mg (146 µmol, 3.0 eq.) (4-pentanoyloxybenzyl)-(4-decanoyloxybenzyl)-*N,N*-di*iso*propylaminophosphoramidite and 0.29 mL (133 µmol, 0.25 mol/L, 1-2 eq.) DCI-activator were used followed by oxidation with 30.0 µL (170 µmol, 5.5 m/L in decane, 3.5 eq.) tert-butylhydroperoxide. The crude product was then purified according to the general procedure 6 (variant B). Vield: 17.4 mg (16.8 µmol, 34%) as a colourless cotton.

### Examples

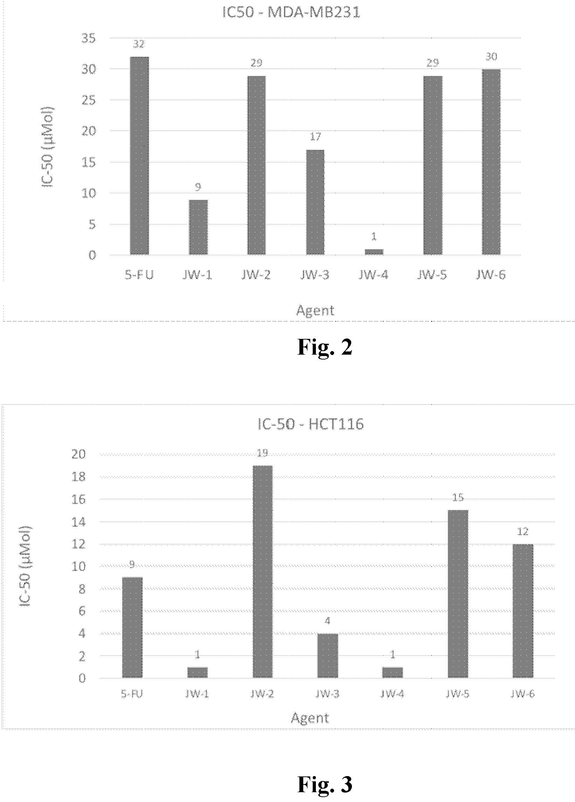

In a first approach, six different JW-substances (JW-1, JW-2, JW-3, JW-4, JW-5, JW-6) having the general structure according to formula 1b above with acyloxybenzyl-groups of different lengths (from 8 to 13 carbon atoms) in three different concentrations (25, 12.5 and 6.25 µmol/l) were investigated. As a comparison, 5-FU was used in the same concentrations. The investigated cell line was a triple-negative breast carcinoma (MDA-MB231), see Fig. 2. The treatment of Triple Negative Breast Cancer is not well standardised, however, one of the treatment options includes 5-FU (Yin L, Duan JJ, Bian XW, Yu SC. Triple-negative breast cancer molecular subtyping and treatment progress. Breast Cancer Res. 2020 Jun 9;22(1):61. doi: 10.1186/s13058-020-01296-5. PMID: 32517735; PMCID: PMC7285581). Hence this particular cell line was chosen because of its molecular characteristics.

**Table 1. Residues R^{A1} and R^{B1} of six different symmetrically masked 5-Fluorouracil nucleoside triphosphate prodrugs according to the invention.**

| **Compound** | **R^{A1}** | **R^{B1}** |
|---|---|---|
| JW-1 | C₈ alkyl | C₈ alkyl |
| JW-2 | C₉ alkyl | C₉ alkyl |
| JW-3 | C₁₀ alkyl | C₁₀ alkyl |
| JW-4 | C₁₁ alkyl | C₁₁ alkyl |
| JW-5 | C₁₂ alkyl | C₁₂ alkyl |
| JW-6 | C₁₃ alkyl | C₁₃ alkyl |

Initially, clear cytotoxic effects were observed for all JW substances. In terms of effectiveness, they are approximately equivalent to 5-FU with the exception of JW-1 and JW-4, the substances with 8 and 11 carbons in the alkyl residue chain. They show significantly stronger cytotoxicity, which can be seen especially in the IC-50 values. IC-50 values are about 3 to 10 times lower.

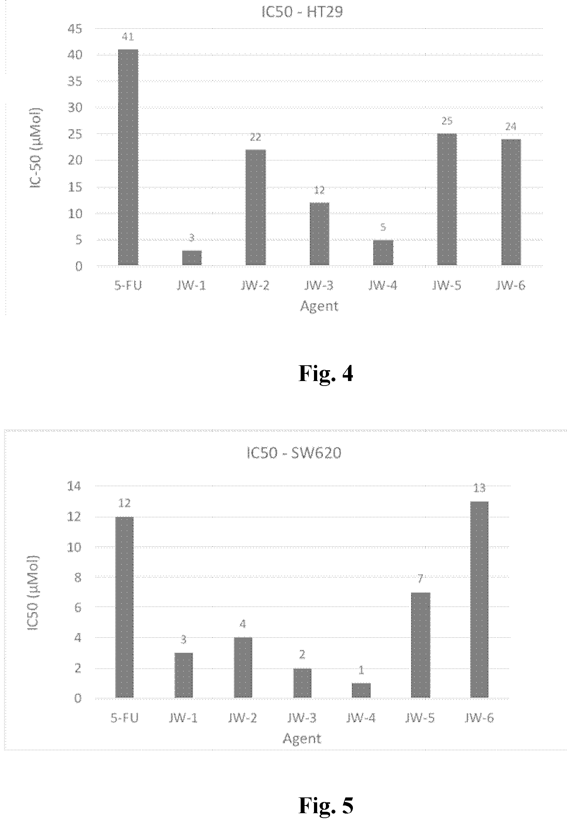

The experiment was also performed for colon carcinoma cell lines HCT116, HT29, and SW620, with similar results as with the breast cancer cell line (see Fig. 3-5).

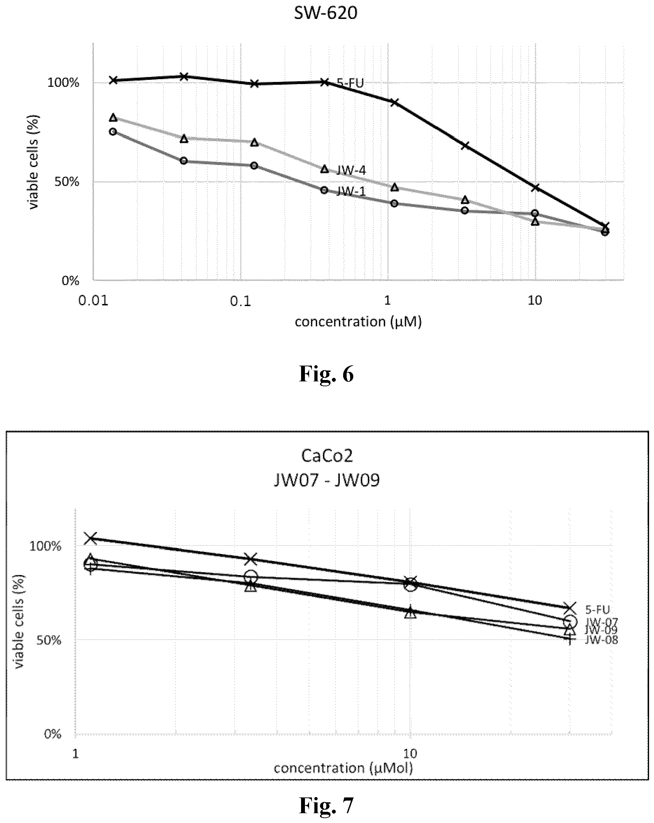

Further investigations were performed in relation these two substances (JW-1 and JW-4). The same procedure as in the first experiments was used, but with a wider concentration range in order to examine the onset of cytotoxicity and the therapeutic range more closely. Thus, JW-1, JW-4 and 5-FU were applied to the cells (colon carcinoma cell line SW620) in the concentrations of 30 µmol in triple dilution up to 0.014 µmol and then examined according to the same scheme. Once again, JW-1 and JW-4 were found to have IC-50 values at least as low as 5-FU (Fig. 6). A major advantage is, however, their increased therapeutic range. The cytotoxicity threshold is much lower than that of 5-FU. In the case of 5-FU, first cytotoxic effects can be detected between 1 and 3 µmol. In comparison, the threshold for JW-1 and JW-4 is below 1 µmol.

Three further JW-substances (JW-7, JW-8, JW-9) having the general structure according to formula 1b above with acyloxybenzyl-groups of different lengths (from 4 to 17 carbon atoms), but being asymmetrically substituted (i.e. having different groups R^{A1} and R^{B1}), in four different concentrations (1.11, 3.33, 10 and 30 µmol/l) were investigated. As a comparison, 5-FU was used in the same concentrations. The investigated cell lines were the colon cancer cell lines CaCo2 and HT29.

The experiments consisted of three steps. On the first day, the cells were planted on 96-well-plates and left for growing and adhering for 24 hours. On the second day, the substances were applied in the known concentrations, diluted in the cell-line specific medium. After an incubation period of 72 hours, XTT-colouring (see, e.g., Roehm NW, Rodgers GH, Hatfield SM, Glasebrook AL, 1991, An improved colorimetric assay for cell proliferation and viability utilizing the tetrazolium salt XTT, J Immunol Methods, 142(2):257-65. doi: 10.1016/0022-1759(91)90114-u) was applied and the absorbance was measured after 6 hours.

**Table 2. Residues R^{A1} and R^{B1} of three further 5-Fluorouracil nucleoside triphosphate prodrugs according to the invention, being asymmetrically substituted with residues R^{A1} and R^{B1}.**

| **Compound** | **R^{A1}** | **R^{B1}** |
|---|---|---|
| JW-7 | C₄ alkyl | C₁₇ alkyl |
| JW-8 | C₇ alkyl | C₉ alkyl |
| JW-9 | C₁₀ alkyl | C₁₂ alkyl |

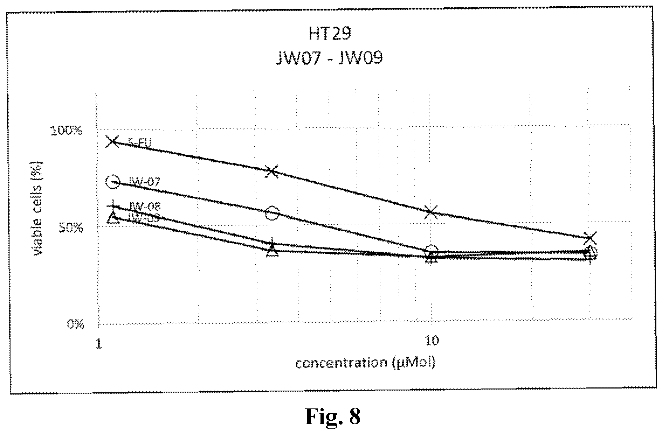

Figures 7 and 8 show that also asymmetrically substituted 5-Fluorouracil nucleoside triphosphate prodrugs of the invention have superior efficacy as regards cytotoxicity against cancer cells in comparison to 5FU. For the HT29 colon cancer cell line (Fig. 8), the compounds according to the invention showed IC₅₀ values of 4-5 µM (JW-07), 2 µM (JW-08) and 1-2 (JW-09), compared to about 15 µM for 5 FU. For CaCo2 cells, IC₅₀ values were higher than those for the HT29 cell line, but nevertheless lower for the compounds of the invention in comparison to 5FU.

## Claims

1. A 5-Fluorouracil nucleoside triphosphate prodrug having the following general formula I 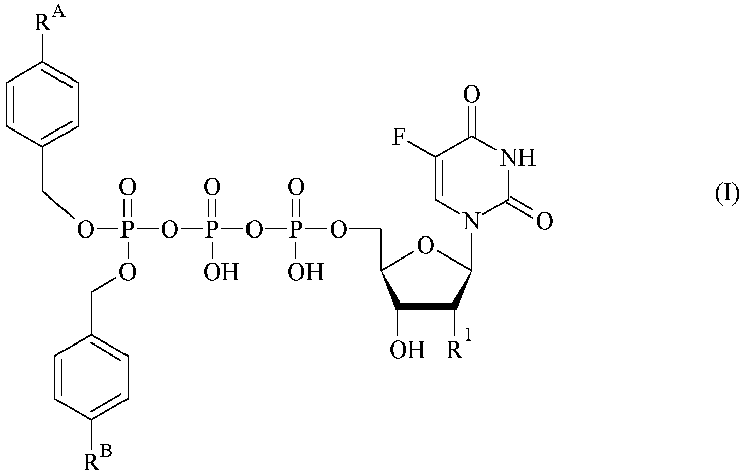 or a pharmaceutically acceptable salt thereof, wherein
R¹ is H or OH,
R^{A} is H or OC(X)R^{A1}, wherein X is O, S or NH,
R^{B} is H or OC(X)R^{B1}, wherein X is O, S or NH,
R^{A} and R^{B} are not both H, and
R^{A1} and R^{B1} are, independently from each other, a substituted or unsubstituted cyclic, acyclic, linear or branched aliphatic or heteroaliphatic residue, or a substituted or unsubstituted aromatic or heteroaromatic residue.

2. The 5-Fluorouracil nucleoside triphosphate prodrug according to claim 1, wherein
R^{A1} and R^{B1} are, independently from each other,
i. a substituted or unsubstituted cyclic, acyclic, linear or branched C_{1―20} aliphatic or C_{1―20} heteroaliphatic residue, or a substituted or unsubstituted C_{5―20} aromatic or C_{3―20} heteroaromatic residue, or
ii. a substituted or unsubstituted cyclic, acyclic, linear or branched C_{1―10} aliphatic or C_{1―10} heteroaliphatic residue, or a substituted or unsubstituted C_{5―12} aromatic or C_{3―12} heteroaromatic residue, or
iii. selected from the group consisting of substituted or unsubstituted C_{1―20} alkyl, substituted or unsubstituted C_{2―20} alkenyl, substituted or unsubstituted C_{2―20} alkynyl, substituted or unsubstituted C_{4―20} alkenynyl, substituted or unsubstituted C_{3―20} cycloalkyl, substituted or unsubstituted C_{3―20} cycloalkenyl, substituted or unsubstituted C_{5―20} cycloalkynyl, substituted or unsubstituted C_{5―20} cycloalkenynyl, substituted or unsubstituted C_{1―20} heteroalkyl, substituted or unsubstituted C_{2―20} heteroalkenyl, substituted or unsubstituted C_{2―20} heteroalkynyl, substituted or unsubstituted C_{4―20} heteroalkenynyl, substituted or unsubstituted C_{5―24} aryl, substituted or unsubstituted C_{3―24} heteroaryl, preferably C_{1―20} alkyl or C_{1―20} alkenyl.

3. The 5-Fluorouracil nucleoside triphosphate prodrug according to one of the preceding claims, wherein R^{A} and R^{B} are identical.

4. The 5-Fluorouracil nucleoside triphosphate prodrug according to one of claims 1 or 0, wherein R^{A} and R^{B} are different from each other.

5. The 5-Fluorouracil nucleoside triphosphate prodrug according to one of the preceding claims, wherein R¹ is H, X is O, R^{A1} is C₅₋₁₅ alkyl, preferably C₈₋₁₃ alkyl, and R^{B1} is C_{5-C15} alkyl, preferably C₈₋₁₃ alkyl.

6. The 5-Fluorouracil nucleoside triphosphate prodrug according to claim5, wherein R^{A1} is C₈ alkyl or C₁₁ alkyl, and R^{B1} is C₈ alkyl or C₁₁ alkyl.

7. The 5-Fluorouracil nucleoside triphosphate prodrug according to claim 6, wherein R^{A1} and R^{B1} are identical.

8. The 5-Fluorouracil nucleoside triphosphate prodrug according to one of the preceding claims for use as a medicament.

9. The 5-Fluorouracil nucleoside triphosphate prodrug according to one of claims 1 to 7 for use as a cancer medicament, preferably as cancer medicament for treating colon carcinoma, breast cancer, esophageal cancer, head and neck cancer, stomach cancer, anal cancer or squamous cell carcinoma.

10. The 5-Fluorouracil nucleoside triphosphate prodrug according to claim 9, for use as a medicament for the treatment of colon carcinoma or triple negative mamma carcinoma.

11. Pharmaceutical composition or dosage form comprising a 5-Fluorouracil nucleoside triphosphate prodrug according to one of claims 1 to 7 and a pharmaceutically acceptable carrier.

12. Pharmaceutical composition or dosage form, being or comprising a liposome or liposome formulation comprising a 5-Fluorouracil nucleoside triphosphate prodrug according to one of claims 1 to 7.
